# EUROPEAN PATENT APPLICATION

(11) **EP 4 834 948 A1**
(43) Date of publication of application: **04.11.2026**
(21) Application number: 24911543.7
(22) Date of filing: 27.12.2024
(51) Int. Cl.: C07K 19/00, C12N 15/62, C12N 15/85, A61K 38/20, A61K 38/17, A61K 47/68, A61P 35/00

(54) **FUSION ANTIBODY AND USE THEREOF**

(30) Priority: 28.12.2023 CN 202311839194
(71) Applicant: Yichen Therapeutics Limited, Wan Chai, Hong Kong (HK)
(72) Inventor: WANG, Feng, Nantong, Jiangsu 226002 (CN); ZHANG, Yuhan, Nantong, Jiangsu 226002 (CN)
(74) Representative: Studio Torta S.p.A.
(86) International application number: PCT/CN2024/143370
(87) International publication number: WO 2025/140627

(57) **Abstract**

The invention provides a fusion antibody and use thereof. The fusion antibody comprises: (a) IL-15; (b) IL-15Ra; and (c) an antibody targeting a therapeutically relevant cell-surface antigen, wherein the antibody comprises a heavy-chain variable region VH, a heavy-chain constant region CH1, a light-chain variable region VL, and a light-chain constant region CL. IL-15/IL-15Ra is located between the C-terminus of the heavy-chain variable region VH and the N-terminus of the heavy-chain constant region CH1, and IL-15/IL-15Ra is located between the C-terminus of the light-chain variable region VL and the N-terminus of the light-chain constant region CL. The fusion antibody can prolong half-life and can exploit antibody targeting to specifically reach the site of action, thereby conferring special cell-targeting capability on IL-15. Receptor activation occurs only in environments with a high receptor density of IL-2βγ, thereby reducing the systemic toxicity of the IL-15/IL-15Ra complex.

## Description

### Cross-reference to related application

This application is based on the Chinese application No. 202311839194.X, filed on December 28, 2023, and claims its priority right. The disclosed content of that Chinese application is again incorporated by reference into this application in its entirety.

### Technical Field

The invention relates to the field of genetic engineering, and in particular to a fusion antibody and use thereof.

### Background

Cytokines are proteins used for signal transduction in living organisms. Cytokines can be produced by many cell types and act on neighboring cells or on the entire system, playing important roles in innate and adaptive immunity.

Studies have found that many cytokines can stimulate immune effector cells and enhance cytotoxicity. This property has led to the use of cytokines in tumor therapy. Interleukin-15 (IL-15) is a cytokine protein in the same family as interleukin-2 (IL-2) and was discovered in 1994. IL-15 shares many biological activities with IL-2, including stimulating the proliferation and activation of T cells and NK cells, inducing immunoglobulin synthesis by B cells, and supporting the differentiation of cytotoxic effector cells.

IL-15 has a structure similar to that of IL-2 and shares the IL2Rβ and IL2Rγ receptors with IL-2. IL-15Ra is a transmembrane protein having high affinity for IL-15. The complex formed by IL-15 and IL-15Ra (IL-15 superagonist) can present IL-15 to the surface of cells expressing IL-2Rβγ, thereby activating proliferation and activation of NK cells and T cells. Unlike IL-2, IL-15 does not lead to activation of regulatory T cells (Treg) and does not mediate activation-induced cell death (AICD), but instead suppresses IL-2-induced AICD. This indicates that IL-15 may be a better choice than IL-2 for tumor therapy.

The antitumor effect of IL-15 mainly occurs through promoting proliferation and activation of CD8+ T cells and NK cells. In multiple experimental animal tumor models (LA795 lung adenocarcinoma, melanoma (B16, B78-H1), MC38 colon cancer, liver cancer, and lymphoma), IL-15 treatment promoted tumor regression, reduced metastasis, and improved survival. However, due to its low molecular weight and short half-life, IL-15 has a high renal clearance rate, making multiple daily injections inconvenient. Thus, the use of recombinant IL-15 alone is also limited in tumor therapy.

Current clinical research drugs commonly use PEGylation or Fc fusion to increase cytokine half-life. Although half-life is prolonged, toxicity due to poor targeting remains a problem. In clinical development, N-803 is currently the most advanced IL-15 fusion protein; however, its poor tumor targeting and superagonist properties impose significant restrictions on its indications and administration routes in tumor therapy.

Therefore, there is a clear need to develop an IL-15 fusion protein having a long half-life, specific tumor therapeutic effect, and low immunotoxicity.

### Summary of the Invention

A primary object of the invention is to provide a fusion antibody and use thereof, so as to solve the problem that IL-15 fusion proteins in the prior art lack tumor cell-targeting or tumor microenvironment (TME)-targeting.

According to a first aspect of the invention, a fusion antibody is provided. The antibody comprises IL-15, IL-15Ra, and an antibody targeting a therapeutically relevant cell-surface antigen. a heavy-chain variable region VH, a heavy-chain constant region CH1, a light-chain variable region VL, and a light-chain constant region CL; wherein IL-15 is located between the C-terminus of VH and the N-terminus of CH1 of the antibody, and IL-15Ra is located between the C-terminus of VL and the N-terminus of CL of the antibody; or IL-15 is located between the C-terminus of VL and the N-terminus of CL of the antibody, and IL-15Ra is located between the C-terminus of VH and the N-terminus of CH1 of the antibody.

Further, IL-15 is fused directly or via a linker between the C-terminus of VH and the N-terminus of CH1 of the antibody, and IL-15Ra is fused directly or via a linker between the C-terminus of VL and the N-terminus of CL of the antibody; or IL-15 is fused directly or via a linker between the C-terminus of VL and the N-terminus of CL of the antibody, and IL-15Ra is fused directly or via a linker between the C-terminus of VH and the N-terminus of CH1 of the antibody.

Further, the C-terminus of CH1 may contain an Fc fragment, and the C-terminus of CL may contain or omit an Fc fragment.

Further, IL-15 has an amino acid sequence as set forth in SEQ ID NO: 12 or SEQ ID NO: 81, or an amino acid sequence having more than 80%, more preferably more than 90%, even more preferably more than 95% homology thereto.

Further, IL-15Ra is full-length IL-15Ra or the Sushi domain of IL-15Ra; preferably, IL-15Ra has an amino acid sequence as set forth in SEQ ID NO: 13 or SEQ ID NO: 14, or an amino acid sequence having more than 80%, more preferably more than 90%, even more preferably more than 95% homology thereto.

Further, the therapeutically relevant cell-surface antigen may be an immune checkpoint protein or a tumor antigen.

Further, the therapeutically relevant cell-surface antigen includes, but is not limited to, any one of the following: PD1, PDL1, B7H3, PSMA, Nectin-4, CD19, BCMA, CD22, CD20, GPCR5D, CD21, CD81, CD40, CD79, CD80, CD86, ICAM-1 (CD54), CD11a, CD18, CD45, GPC3, HER2, EGFR, GCN4, Tim3, CLL1, Trop2, Claudin18.2, Claudin6, Muc1, Muc16, or GIST.

Further, the antibody targeting the therapeutically relevant cell-surface antigen comprises an anti-PDL1 antibody having HCDR1, HCDR2, HCDR3 as set forth in SEQ ID NOs: 15-17 and LCDR1, LCDR2, LCDR3 as set forth in SEQ ID NOs: 18-20; or a VH sequence as set forth in SEQ ID NO: 21 and a VL sequence as set forth in SEQ ID NO: 22; or a heavy-chain amino acid sequence as set forth in SEQ ID NO: 23 and a light-chain amino acid sequence as set forth in SEQ ID NO: 24.

Further, the antibody targeting the therapeutically relevant cell-surface antigen comprises an anti-PD1 antibody having HCDR1, HCDR2, HCDR3 as set forth in SEQ ID NOs: 120-122, SEQ ID NOs: 126-128, or SEQ ID NOs: 132-134, and LCDR1, LCDR2, LCDR3 as set forth in SEQ ID NOs: 123-125, SEQ ID NOs: 129-131, or SEQ ID NOs: 135-137; or a VH sequence as set forth in SEQ ID NO: 138 and a VL sequence as set forth in SEQ ID NO: 139; or a heavy-chain amino acid sequence as set forth in SEQ ID NO: 140 or 142 and a light-chain amino acid sequence as set forth in SEQ ID NO: 141 or 143.

Preferably, the antibody targeting the therapeutically relevant cell-surface antigen comprises an anti-B7H3 antibody having HCDR1, HCDR2, HCDR3 as set forth in SEQ ID NOs: 25-27 and LCDR1, LCDR2, LCDR3 as set forth in SEQ ID NOs: 28-30; or a VH sequence as set forth in SEQ ID NO: 31 or 118 and a VL sequence as set forth in SEQ ID NO: 32 or 119; or a heavy-chain amino acid sequence as set forth in SEQ ID NO: 33 and a light-chain amino acid sequence as set forth in SEQ ID NO: 34.

Preferably, the antibody targeting the therapeutically relevant cell-surface antigen comprises an anti-PSMA antibody having HCDR1, HCDR2, HCDR3 as set forth in SEQ ID NOs: 35-37 and LCDR1, LCDR2, LCDR3 as set forth in SEQ ID NOs: 38-40; or a VH sequence as set forth in SEQ ID NO: 41 and a VL sequence as set forth in SEQ ID NO: 42; or a heavy-chain amino acid sequence as set forth in SEQ ID NO: 43 and a light-chain amino acid sequence as set forth in SEQ ID NO: 44.

Further, the antibody targeting the therapeutically relevant cell-surface antigen comprises an Fc fragment; preferably, the Fc fragment is selected from Fc fragments of human IgG1, IgG2, IgG3, or IgG4.

Further, the linker peptide is a cleavable linker peptide or a non-cleavable linker peptide; preferably, each of the linker peptides is independently selected from [GGGGS]n(n=1-6), EAAAK (SEQ ID NO:79), EAAAKEAAAK (SEQ ID NO:80), GGGGSIPVSLRSGGGGGSG (SEQ ID NO:65) or GGGGSIPVSLRSGGGSG (SEQ ID NO:62), GGGGSG (SEQ ID NO:145), GGGGSGGGGSG (SEQ ID NO:146), GGGGSGGGGSGGGGSG (SEQ ID NO:64); more preferably, the linker peptide is GGGGS (SEQ ID NO:144), GGGGSG (SEQ ID NO:145), GGGGSGGGGS (SEQ ID NO:63), GGGGSGGGGSG (SEQ ID NO:146), GGGGSGGGGSGGGGSG (SEQ ID NO:64), GGGGSIPVSLRSGGGGGSG (SEQ ID NO:65) or GGGGSIPVSLRSGGGSG (SEQ ID NO:62).

Further, the fusion antibody comprises a heavy chain and a light chain having any of the following groups of amino acid sequences, respectively: 1) SEQ ID NO: 1 and SEQ ID NO: 2; 2) SEQ ID NO: 3 and SEQ ID NO: 4; 3) SEQ ID NO: 1 and SEQ ID NO: 5; 4) SEQ ID NO: 1 and SEQ ID NO: 6; 5) SEQ ID NO: 7 and SEQ ID NO: 8; 6) SEQ ID NO: 9 and SEQ ID NO: 10; 7) SEQ ID NO: 5 and SEQ ID NO: 11; 8) SEQ ID NO: 45 and SEQ ID NO: 46; 9) SEQ ID NO: 57 and SEQ ID NO: 58; 10) SEQ ID NO: 59 and SEQ ID NO: 60; 11) SEQ ID NO: 57 and SEQ ID NO: 60; 12) SEQ ID NO: 67 and SEQ ID NO: 68; 13) SEQ ID NO: 69 and SEQ ID NO: 70; 14) SEQ ID NO: 71 and SEQ ID NO: 72; 15) SEQ ID NO: 73 and SEQ ID NO: 74; 16) SEQ ID NO: 75 and SEQ ID NO: 76; 17) SEQ ID NO: 77 and SEQ ID NO: 78; 18) SEQ ID NO: 82 and SEQ ID NO: 8; 19) SEQ ID NO: 83 and SEQ ID NO: 84; 20) SEQ ID NO: 85 and SEQ ID NO: 84; 21) SEQ ID NO: 83 and SEQ ID NO: 86; 22) SEQ ID NO: 85 and SEQ ID NO: 86; 23) SEQ ID NO: 88 and SEQ ID NO: 89; 24) SEQ ID NO: 90 and SEQ ID NO: 91; 25) SEQ ID NO: 92 and SEQ ID NO: 93; 26) SEQ ID NO: 94 and SEQ ID NO: 95; 27) SEQ ID NO: 96 and SEQ ID NO: 97; 28) SEQ ID NO: 98 and SEQ ID NO: 99; 29) SEQ ID NO: 102 and SEQ ID NO: 103; 30) SEQ ID NO: 106 and SEQ ID NO: 107; 31) SEQ ID NO: 108 and SEQ ID NO: 107; 32) SEQ ID NO: 112 and SEQ ID NO: 113; 33) SEQ ID NO: 112 and SEQ ID NO: 114; 34) SEQ ID NO: 115 and SEQ ID NO: 116; 35) SEQ ID NO: 115 and SEQ ID NO: 117.

To achieve the above objective, according to a second aspect of the present invention, a DNA molecule is provided, which encodes the fusion antibody described above.

To achieve the above objective, according to a third aspect of the present invention, a recombinant plasmid is provided, which is ligated to the DNA molecule described above.

To achieve the above objective, according to a fourth aspect of the present invention, a host cell is provided, which is transformed with the recombinant plasmid described above.

Further, the host cell comprises a prokaryotic cell or a eukaryotic cell.

To achieve the above objective, according to a fifth aspect of the present invention, there is provided a use of the fusion antibody described above in the manufacture of a medicament for the prophylaxis and/or treatment of cancer.

Further, the cancer comprises any one of the following: lung cancer, melanoma, colon cancer, rectal cancer, liver cancer, breast cancer, lymphoma, prostate cancer, or hematological cancer.

To achieve the above objective, according to a sixth aspect of the present invention, a medicament is provided, comprising the fusion antibody described above.

Further, the medicament further comprises an immune checkpoint inhibitor for combined use with the fusion antibody, wherein the immune checkpoint inhibitor comprises any one or more of the following: anti-PDL1, anti-CTLA4, anti-41BB, or anti-LAG3.

By applying the technical solution of the present invention, the IL-15 domain or IL-15Ra domain is fused to between VH-CH1 or VL-CL of the targeting antibody through a linker peptide, respectively, to form a targeting fusion antibody in which IL-15 and IL-15Ra are paired. This specific fusion method: 1) fully exposes the N-terminus of the targeting antibody, making full use of the targeting function of the antibody to specifically enrich IL-15 to the target site; 2) confers IL-15 with a half-life consistent with that of the targeting antibody; 3) attenuates the binding activity of IL-15 to IL-2Rβγ, significantly reducing the systemic toxicity of IL-15 while maintaining the receptor-activating function of IL-15 in an environment with high-density IL-2Rβγ.

### Brief Description of the Drawings

The accompanying drawings, which form a part of the specification, are provided to further explain the present invention. The exemplary embodiments of the present invention and their descriptions are used to explain the present invention and do not constitute an improper limitation thereof. In the drawings:
Figure 1 shows the SDS-PAGE gel of the fusion antibody purified in Example 2 of the present application;
Figure 2 shows the SEC analysis of the fusion antibody purified in Example 2 of the present application;
Figure 3 shows the thermodynamic stability profiles of fusion antibodies FuAb1, FuAb5, FuAb6, FuAb17, FuAb18 and FuAb19 in Example 4;
Figure 4 shows the results of ELISA detection of the binding of different fusion antibodies to the antigen PDL1 in Example 5;
Figure 5 shows the results of ELISA detection of the binding of different fusion antibodies to the antigen PDL1 in Example 5, where FuAb-C represents the enzyme-digested fusion antibody;
Figure 6 shows the results of ELISA detection of the binding of different fusion antibodies to the antigens B7H3, PSMA, IL-15Ra, and IL-2Rβγ in Example 5;
Figure 7 shows the results of ELISA detection of the binding of different fusion antibodies to the antigens B7H3, IL-15Ra, and IL-2Rβγ in Example 5;
Figure 8 shows the results of ELISA detection of the binding of different fusion antibodies to the antigens B7H3 and IL-2Rβγ in Example 5;
Figure 9 shows the results of ELISA detection of the binding of different fusion antibodies to the antigen PD1 in Example 5;
Figure 10 shows the results of ELISA detection of the binding of different fusion antibodies to the antigen IL-2Rβγ in Example 5;
Figure 11 shows the results of binding activity assays of different fusion antibodies to cells expressing different antigens in Example 5;
Figure 12 shows the results of binding activity assays of different fusion antibodies to cells expressing different antigens in Example 5, where FuAb-C represents the enzyme-digested fusion antibody;
Figure 13 shows the results of binding activity assays of different fusion antibodies to Mo7e cells in Example 5;
Figure 14 shows the proliferation-promoting activity of different fusion antibodies on CTLL2 cells in Example 6;
Figure 15 shows the proliferation-promoting activity of different fusion antibodies on CTLL2 cells in Example 6;
Figure 16 shows the proliferation-promoting activity of different fusion antibodies on Mo7e cells in Example 6;
Figure 17 shows the proliferation-promoting activity of different fusion antibodies on Mo7e cells in Example 6;
Figure 18 shows the proliferation-promoting activity of different fusion antibodies on PBMC cells in Example 6;
Figure 19 shows the proliferation-promoting activity of different fusion antibodies on NK92 cells in Example 6;
Figure 20 shows the effects of different fusion antibody treatments on tumor volume and mouse body weight in the MC38 mouse tumor model in Example 7;
Figure 21 shows the effects of PD1-targeted fusion antibodies on tumor volume and mouse body weight in the MC38 mouse tumor model in Example 7;
Figure 22 shows the effects of PD1-targeted fusion antibodies on cell population changes in the MC38 mouse tumor model in Example 7;
Figure 23 shows the effects of different doses of fusion antibody FuAb1 on tumor volume and mouse body weight in the MC38 mouse tumor model in Example 8;
Figure 24 shows the flow cytometric analysis of cell populations in peripheral blood samples from MC38 mouse tumor models treated with fusion antibodies in Example 9;
Figure 25 shows the flow cytometric analysis of cell populations in spleen samples from MC38 mouse tumor models treated with fusion antibodies in Example 9;
Figure 26 shows the flow cytometric analysis of cell populations in tumor tissue samples from MC38 mouse tumor models treated with fusion antibodies in Example 9;
Figure 27 shows the effects of different doses of fusion antibodies on tumor volume and mouse body weight in the MC38-hB7H3 mouse tumor model in Example 10;
Figure 28 shows the effects of fusion antibody FuAb6 alone or in combination with aCTLA4 at different doses on tumor volume and mouse body weight in the mouse tumor model in Example 10;
Figure 29 shows the effects of different doses of fusion antibodies on mouse tumor volume in the MC38-hB7H3 mouse tumor model in Example 11;
Figure 30 shows the effects of different doses of fusion antibodies on mouse body weight in the MC38-hB7H3 mouse tumor model in Example 11;
Figure 31 shows a schematic diagram of the fusion antibody obtained by fusing IL-15 with the heavy chain (first chain) of the antibody and IL-15Ra with the light chain (second chain) of the antibody or the light chain with an Fc region at the C-terminus;

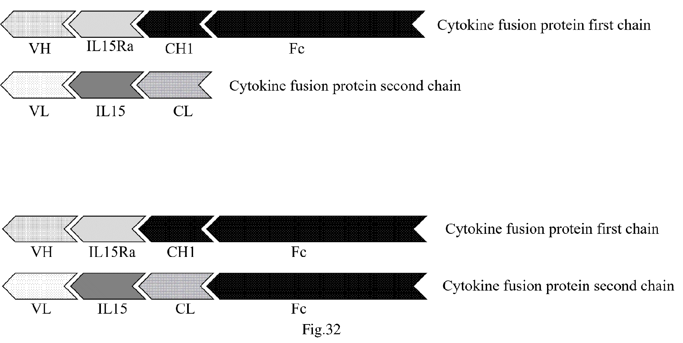

Figure 32 shows a schematic diagram of the fusion antibody obtained by fusing IL-15 with the light chain (second chain) of the antibody or the light chain with an Fc region at the C-terminus and IL-15Ra with the heavy chain (first chain) of the antibody.

### Detailed Description

It should be noted that the embodiments in the present application and the features in the embodiments may be combined with each other without conflict. The present invention will be described in detail below with reference to the accompanying drawings and embodiments.

As mentioned in the Background Art, the IL-15 fusion proteins in the prior art have the following problems: 1) lack of targeting to tumor cells or the tumor microenvironment; 2) relatively short half-life in pharmacokinetic performance, unable to match therapeutic antibodies; 3) strong peripheral side effects, and the safe dose cannot match therapeutic antibodies.

Although the IL-15 fusion protein can form a complex with IL-15Ra and bind the IL-2Rβγ receptor, thereby promoting the proliferation and activation of NK cells and T cells and exerting an anti-tumor effect. However, due to the short half-life and the poor cell targeting, the superagonist activity of the IL-15/IL-15Ra complex tends to induce systemic immune toxicity. Accordingly, multiple administrations are required, and unavoidable immune toxicity remains intractable in anti-tumor therapeutic applications of IL-15. The present application intends to embed IL-15 and IL-15Ra into an antibody to obtain a fusion antibody with high cell-targeting for tumor prevention and treatment.

In a first representative embodiment, the invention provides a fusion antibody comprising: a) IL-15; b) IL-15Ra; and c) an antibody targeting a therapeutically relevant cell-surface antigen, wherein the antibody comprises a heavy-chain variable region VH, a heavy-chain constant region CH1, a light-chain variable region VL, and a light-chain constant region CL; wherein IL-15 is located between the C-terminus of VH and the N-terminus of CH1 of the antibody, and IL-15Ra is positioned between the C-terminus of VL and the N-terminus of CL of the antibody; or IL-15 is positioned between the C-terminus of VL and the N-terminus of CL of the antibody, and IL-15Ra is positioned between the C-terminus of VH and the N-terminus of CH1 of the antibody.

The IL-15/IL-15Ra complex can bind IL-2Rβγ, thereby activating proliferation and activation of NK cells and T cells for anti-tumor therapy. In the present application, IL-15 and IL-15Ra are respectively embedded between VH-CH1 or VL-CL. Through the targeting ability of the antibody toward the cell-surface antigen, the IL-15/IL-15Ra complex can specifically accumulate in the tumor microenvironment and specifically kill tumors without affecting the antibody's specific antigen recognition, thereby avoiding systemic immune toxicity caused by excessive activation of NK cells. At the same time, embedding IL-15 and IL-15Ra into the antibody prolongs the half-life of the IL-15/IL-15Ra complex and facilitates practical pharmaceutical use of IL-15.

IL-15 and IL-15Ra can be embedded between VL-CL or VH-CH1, and the connection between IL-15, IL-15Ra and the antibody may be direct or through a linker peptide. The flexibility of the linker allows the molecules connected at its two ends to maintain their inherent biological activities.

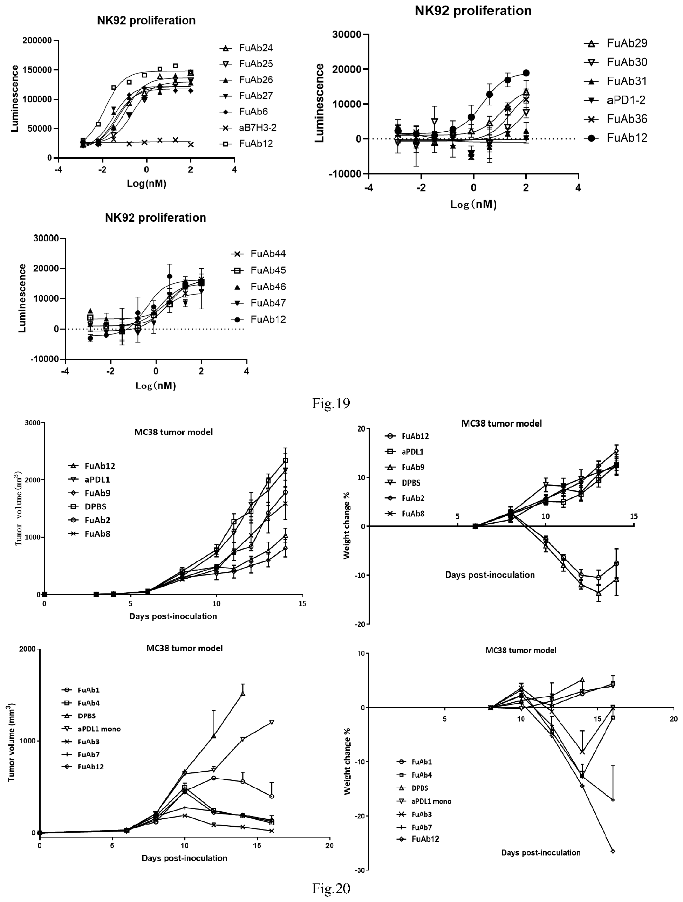

As shown in Figure 19, in a preferred embodiment, IL-15 is directly embedded between the C-terminus of VH and the N-terminus of CH1 of the antibody, and IL-15Ra is embedded between the C-terminus of VL and the N-terminus of CL of the antibody.

In a preferred embodiment, IL-15Ra is embedded via a linker between the C-terminus of VH and the N-terminus of CH1 of the antibody; IL-15Ra is embedded via a linker between the C-terminus of VL and the N-terminus of CL of the antibody.

In a preferred embodiment, IL-15 is directly embedded between the C-terminus of VH and the N-terminus of CH1 of the antibody, and IL-15Ra is embedded via a linker between the C-terminus of VL and the N-terminus of CL of the antibody.

In a preferred embodiment, IL-15Ra is embedded via a linker between the C-terminus of VH and the N-terminus of CH1 of the antibody; IL-15Ra is directly embedded between the C-terminus of VL and the N-terminus of CL of the antibody.

As shown in Figure 20, in a preferred embodiment, IL-15 is directly embedded between the C-terminus of VL and the N-terminus of CL of the antibody, and IL-15Ra is directly embedded between the C-terminus of VH and the N-terminus of CH1 of the antibody.

In a preferred embodiment, IL-15 is embedded via a linker between the C-terminus of VL and the N-terminus of CL of the antibody, and IL-15Ra is embedded via a linker between the C-terminus of VH and the N-terminus of CL of the antibody.

In a preferred embodiment, IL-15 is directly embedded between the C-terminus of VL and the N-terminus of CL of the antibody, and IL-15Ra is embedded via a linker between the C-terminus of VH and the N-terminus of CH1 of the antibody.

In a preferred embodiment, IL-15 is embedded via a linker between the C-terminus of VL and the N-terminus of CL of the antibody, and IL-15Ra is directly embedded between the C-terminus of VH and the N-terminus of CH1 of the antibody.

Any light-chain having a VL-CL structure or any heavy-chain having a VH-CH1 structure is suitable for the present application. In a preferred embodiment, the C-terminus of CH1 contains Fc, and the C-terminus of CL may contain Fc or may omit Fc.

Any IL-15 having anti-tumor activity of promoting the proliferation and activation of CD8+ T cells and NK cells is suitable for the present application. In a preferred embodiment, IL-15 has an amino acid sequence as shown in SEQ ID NO: 12 or SEQ ID NO: 81, or an amino acid sequence having more than 80%, more preferably more than 90%, and even more preferably more than 95% homology with the amino acid sequence as shown in SEQ ID NO: 12 or SEQ ID NO: 81.
SEQ ID NO:12 hIL15 
NWVNVISDLKKIEDLIQSMHIDATLYTESDVHPSCKVTAMKCFLLELQVISLESGDASIHDTVENLI
ILANDSLSSNGNVTESGCKECEELEEKNIKEFLQSFVHIVQMFINTS.
SEQ ID NO:81: hIL15 WT 
NWVNVISDLKKIEDLIQSMHIDATLYTESDVHPSCKVTAMKCFLLELQVISLESGDASIHDTVENLI
ILANNSLSSNGNVTESGCKECEELEEKNIKEFLQSFVHIVQMFINTS.
 Any IL-15Ra that binds to IL-15 to form an anti-tumor IL-15Ra is suitable for the present application. In a preferred embodiment, IL-15Ra is full-length IL-15Ra or the Sushi domain of IL-15Ra. The Sushi domain of IL-15Ra is a domain with a specific structure, located at the N-terminus of IL-15Ra, consisting of a series of sequences repeating about 60 amino acids, and plays an important role in cell signal transduction and immune response. Preferably, IL-15Ra has an amino acid sequence as shown in SEQ ID NO: 13 or SEQ ID NO: 14, or an amino acid sequence having more than 80%, more preferably more than 90%, and even more preferably more than 95% homology with the amino acid sequence as shown in SEQ ID NO: 13 or SEQ ID NO: 14.
SEQ ID NO:13: hIL15Ra 
ITCPPPMSVEHADIWVKSYSLYSRERYICNSGFKRKAGTSSLTECVLNKATNVAHWTTPSLKCIR
DPALVHQRPAPPSTVTTAGVTPQPESLSPSGKEPAASSPSSNNTAATTAAIVPGSQLMPSKSPSTG
TTEISSHESSHGTPSQTTAKNWELTASASHQPPGVYPQGHSDTT.
SEQ ID NO:14: hIL15RaSushi

ITCPPPMSVEHADIWVKSYSLYSRERYICNSGFKRKAGTSSLTECVLNKATNVAHWTTPSLKCIR.

A therapeutically relevant cell-surface antigen is an antigen on the cell surface that can be targeted for IL-15-mediated treatment of disease. In a preferred embodiment, therapeutically relevant cell-surface antigen is an immune checkpoint protein or a tumor antigen. In a preferred embodiment, the therapeutically relevant cell-surface antigen is selected from, but not limited to, any one of the following: PD1, PDL1, B7H3, PSMA, Nectin-4, CD19, BCMA, CD22, CD20, GPCR5D, CD21, CD81, CD40, CD79, CD80, CD86, ICAM-1 (CD54), CD11a, CD18, CD45, GPC3, HER2, EGFR, Claudin18.2, PD1, GIST, CLL1 or Tim3; preferably, therapeutically relevant cell-surface antigen is selected from any one of the following: PDL1, PD1, B7H3 or PSMA.

Any antibody capable of binding an antigen relevant to IL-15-mediated therapy and capable of accommodating IL-15 or IL-15Ra embedded between VH-CH1 or VL-CL can be used, including monoclonal antibodies and engineered antibodies. The antibody comprises heavy and light chains, each including variable and constant regions. The variable regions respectively contain three complementarity-determining regions (CDRs), which determine specificity and affinity for the antigen.

Any antibody that can bind to an antigen for IL-15-mediated disease therapy and can accommodate IL-15 or IL-15Ra embedded between VH-CH1 or VL-CL is suitable for the present application, including but not limited to monoclonal antibodies and engineered antibodies. The antibody comprises a heavy chain and a light chain, each comprising variable and constant region structures, and the heavy and light chains each have three complementarity-determining regions (CDRs) within the variable region sequences. The CDRs determine the specificity and affinity of the antibody for antigen binding, and their amino acid sequences vary greatly among different antibody molecules.

Different antigens correspond to antibodies with different variable regions. In a preferred embodiment, the antibody targeting a therapeutically relevant cell-surface antigen comprises an anti-PDL1 antibody having heavy-chain complementarity-determining regions HCDR1, HCDR2, HCDR3 as set forth in SEQ ID NOs: 15-17 and light-chain complementarity-determining regions LCDR1, LCDR2, LCDR3 as set forth in SEQ ID NOs: 18-20; or a VH sequence as set forth in SEQ ID NO: 21 and a VL sequence as set forth in SEQ ID NO: 22; or a heavy-chain amino acid sequence as set forth in SEQ ID NO: 23 and a light-chain amino acid sequence as set forth in SEQ ID NO: 24.
aPDL1 HCDR1: DSWIH (SEQ ID NO:15)
aPDL1 HCDR2: WISPYGGSTYYADSVKG(SEQ ID NO:16)
aPDL1 HCDR3: RHWPGGFDY(SEQ ID NO:17)
aPDL1 LCDR1: RASQDVSTAVA(SEQ ID NO:18)
aPDL1 LCDR2: SASFLYS(SEQ ID NO:19)
aPDL1 LCDR3: QQYLYHPAT(SEQ ID NO:20)
aPDL1 VH: 

(SEQ ID NO:21).
aPDL1 VL: 

(SEQ ID NO:22).
aPDL1 heavy-chain: 
EVQLVESGGGLVQPGGSLRLSCAASGFTFSDSWIHWVRQAPGKGLEWVAWISPYGGSTYYADSVKG
RFTISADTSKNTAYLQMNSLRAEDTAVYYCARRHWPGGFDYWGQGTLVTVSSASTKGPSVFPLAPSS
 
KSTSGGTAALGCLVKDYFPEPVTVSWNSGALTSGVHTFPAVLQSSGLYSLSSVVTVPSSSLGTQTYIC
NVNHKPSNTKVDKKVEPKSCDKTHTCPPCPAPPVAGPSVFLFPPKPKDTLMISRTPEVTCVVVDVSHE
DPEVKFNWYVDGVEVHNAKTKPREEQYNSTYRVVSVLTVLHQDWLNGKEYKCKVSNKGLPSSIEKT
ISKAKGQPREPQVYTLPPSRDELTKNQVSLTCLVKGFYPSDIAVEWESNGQPENNYKTTPPVLDSDGS
FFLYSKLTVDKSRWQQGNVFSCSVMHEALHNHYTQKSLSLSPGK(SEQ ID NO:23)
aPDL1 light chain: 
DIQMTQSPSSLSASVGDRVTITCRASQDVSTAVAWYQQKPGKAPKLLIYSASFLYSGVPSRFSGSGSGT
DFTLTISSLQPEDFATYYCQQYLYHPATFGQGTKVEIKRTVAAPSVFIFPPSDEQLKSGTASVVCLLNN
FYPREAKVQWKVDNALQSGNSQESVTEQDSKDSTYSLSSTLTLSKADYEKHKVYACEVTHQGLSSPV
TKSFNRGEC(SEQ ID NO:24)
 In a preferred embodiment, the antibody targeting a therapeutically relevant cell-surface antigen comprises an antibody targeting PD1, having heavy-chain complementarity determining regions HCDR1, HCDR2, and HCDR3 sequences as set forth in SEQ ID NOs: 120-122, and light-chain complementarity determining regions LCDR1, LCDR2, and LCDR3 sequences as set forth in SEQ ID NOs: 123-125; or having heavy-chain CDR sequences as set forth in SEQ ID NOs: 126-128 and light-chain CDR sequences as set forth in SEQ ID NOs: 129-131; or having heavy-chain CDR sequences as set forth in SEQ ID NOs: 132-134 and light-chain CDR sequences as set forth in SEQ ID NOs: 135-137; or having a VH sequence as set forth in SEQ ID NO: 138 and a VL sequence as set forth in SEQ ID NO: 139; or having a heavy-chain amino acid sequence as set forth in SEQ ID NO: 140 and a light-chain amino acid sequence as set forth in SEQ ID NO: 141; or having a heavy-chain amino acid sequence as set forth in SEQ ID NO: 142 and a light-chain amino acid sequence as set forth in SEQ ID NO: 143.
aPD1 HCDR1:AQYMH(SEQ ID NO:120)
aPD1 HCDR2: IINPSGGETGYAQKFQG(SEQ ID NO:121)
aPD1 HCDR3: EGVADGYGLVDV(SEQ ID NO:122)
aPD1 LCDR1: RASQSVSSYLA(SEQ ID NO:123)
aPD1 LCDR2: DASKRAT(SEQ ID NO:124)
aPD1 LCDR3: DQRNNWPLT(SEQ ID NO:125)
aPD1 VH: 
EIVLTQSPATLSLSPGERATLSCRASQSVSSYLAWYQQKPGQAPRLLIYDASKRATGIPARFSGSGSGT
DFTLTISSLEPEDFAVYYCDQRNNWPLTFGGGTKVEIK(SEQ ID NO:138)
aPD1 VL: 
QVQLVQSGAEVKKPGASVKVSCKASGYTFTAQYMHWVRQAPGQGLEWMGIINPSGGETGYAQKFQ
GRVTMTRDTSTSTVYMELSSLRSEDTAVYYCAKEGVADGYGLVDVWGQGTMVTVSS(SEQ ID
NO:139)
aPD1-2 HCDR1: NYYMY(SEQ ID NO:132)
aPD1-2 HCDR2: GINPSNGGTNFNEKFKN(SEQ ID NO:133)
aPD1-2 HCDR3: RDYRFDMGFDY(SEQ ID NO:134)
aPD1-2 LCDR1: RASKGVSTSGYSYLH(SEQ ID NO:135)
aPD1-2 LCDR2: LASYLES(SEQ ID NO:136)
aPD1-2 LCDR3: QHSRDLPLT(SEQ ID NO:137)
aPD1-2 VH: 

(SEQ ID
NO:142)
aPD1-2 VL: 

(SEQ ID NO:143)
aPD1-3 HCDR1: NSGMH(SEQ ID NO:126)
aPD1-3 HCDR2: VIWYDGSKRYYADSVKG(SEQ ID NO:127)
aPD1-3 HCDR3: NDDY(SEQ ID NO:128)
aPD1-3 LCDR1: RASQSVSSYLA(SEQ ID NO:129)
aPD1-3 LCDR2: DASNRAT(SEQ ID NO:130)
aPD1-3 LCDR3: QQSSNWPRT(SEQ ID NO:131)
aPD1-3 VH: 

(SEQ ID NO:140)
aPD1-3 VL: 

(SEQ ID NO:141)
 In a preferred embodiment, the antibody targeting a therapeutically relevant cell-surface antigen comprises an antibody targeting B7H3, having heavy-chain CDR sequences as set forth in SEQ ID NOs: 25-27, and light-chain CDR sequences as set forth in SEQ ID NOs: 28-30; or having a VH sequence as set forth in SEQ ID NO: 31 and a VL sequence as set forth in SEQ ID NO: 32; or having a heavy-chain amino acid sequence as set forth in SEQ ID NO: 33 and a light-chain amino acid sequence as set forth in SEQ ID NO: 34.
aB7H3 HCDR1: NYDIN(SEQ ID NO:25)
aB7H3 HCDR2: WIFPGDGSTQYNEKFKG(SEQ ID NO:26)
aB7H3 HCDR3: QTTATWFAY(SEQ ID NO:27)
aB7H3 LCDR1: RASQSISDYLH(SEQ ID NO:28)
aB7H3 LCDR2: YASQSIS(SEQ ID NO:29)
aB7H3 LCDR3: QNGHSFPLT(SEQ ID NO:30)
aB7H3 VH: 
QVQLQQSGAELVKPGASVKLSCKASGYTFTNYDINWVRQRPEQGLEWIGWIFPGDGSTQYNEKFKG
KATLTTDTSSSTAYMQLSRLTSEDSAVYFCARQTTATWFAYWGQGTLVTVSA(SEQ ID NO:31)
aB7H3 VL: 
DIVMTQSPATLSVTPGDRVSLSCRASQSISDYLHWYQQKSHESPRLLIKYASQSISGIPSRFSGSGSGSD
FTLSINSVEPEDVGVYYCQNGHSFPLTFGAGTKLELK(SEQ ID NO:32)
aB7H3 heavy-chain: 
QVQLQQSGAELVKPGASVKLSCKASGYTFTNYDINWVRQRPEQGLEWIGWIFPGDGSTQYNEKFKG
KATLTTDTSSSTAYMQLSRLTSEDSAVYFCARQTTATWFAYWGQGTLVTVSAASTKGPSVFPLAPSS
KSTSGGTAALGCLVKDYFPEPVTVSWNSGALTSGVHTFPAVLQSSGLYSLSSVVTVPSSSLGTQTYIC
NVNHKPSNTKVDKKVEPKSCDKTHTCPPCPAPPVAGPSVFLFPPKPKDTLMISRTPEVTCVVVDVSHE
DPEVKFNWYVDGVEVHNAKTKPREEQYNSTYRVVSVLTVLHQDWLNGKEYKCKVSNKGLPSSIEKT
ISKAKGQPREPQVYTLPPSRDELTKNQVSLTCLVKGFYPSDIAVEWESNGQPENNYKTTPPVLDSDGS
FFLYSKLTVDKSRWQQGNVFSCSVMHEALHNHYTQKSLSLSPGK(SEQ ID NO:33)
aB7H3 light-chain: 
DIVMTQSPATLSVTPGDRVSLSCRASQSISDYLHWYQQKSHESPRLLIKYASQSISGIPSRFSGSGSGSD
FTLSINSVEPEDVGVYYCQNGHSFPLTFGAGTKLELKKRTVAAPSVFIFPPSDEQLKSGTASVVCLLNN
FYPREAKVQWKVDNALQSGNSQESVTEQDSKDSTYSLSSTLTLSKADYEKHKVYACEVTHQGLSSPV
TKSFNRGEC(SEQ ID NO:34)
aB7H3-2 VH: 
EVQLVQSGAEVKKPGASVKVSCKASGYTFTNYDINWVRQAPGQGLEWIGWIFPGDGSTQYNEKFKG
RATLTTDTSTSTAYMELRSLRSDDTAVYFCARQTTATWFAYWGQGTLVTVSS(SEQ ID NO:118)
aB7H3-2 VL: 
DIVMTQSPLSLPVTPGEPASISCRASQSISDYLHWYLQKPGESPRLLIKYASQSISGIPDRFSGSGSGSDF
TLKISRVEAEDVGVYYCQNGHSFPLTFGAGTKLEIK(SEQ ID NO:119)
 In a preferred embodiment, the antibody targeting a therapeutically relevant cell-surface antigen comprises an antibody targeting PSMA, having heavy-chain CDR sequences as set forth in SEQ ID NOs: 35-37, and light-chain CDR sequences as set forth in SEQ ID NOs: 38-40; or having a VH sequence as set forth in SEQ ID NO: 41 and a VL sequence as set forth in SEQ ID NO: 42; or having a heavy-chain amino acid sequence as set forth in SEQ ID NO: 43 and a light-chain amino acid sequence as set forth in SEQ ID NO: 44.
aPSMA HCDR1: EYTIH(SEQ ID NO:35)
aPSMA HCDR2: NINPNNGGTTYNQKFED(SEQ ID NO:36)
aPSMA HCDR3: GWNFDY(SEQ ID NO:37)
aPSMA LCDR1: KASQDVGTAVD(SEQ ID NO:38)
aPSMA LCDR2: WASTRHT(SEQ ID NO:39)
aPSMA LCDR3: QQYNSYPLT(SEQ ID NO:40)
aPSMA VH: 
EVQLVQSGPEVKKPGATVKISCKTSGYTFTEYTIHWVKQAPGKGLEWIGNINPNNGGTTYNQKFEDK
ATLTVDKSTDTAYMELSSLRSEDTAVYYCAAGWNFDYWGQGTLLTVSS(SEQ ID NO:41)
aPSMA VL: 
DIQMTQSPSSLSTSVGDRVTLTCKASQDVGTAVDWYQQKPGQSPKLLIYWASTRHTGIPSRFSGSGSG
TDFTLTISSLQPEDFADYYCQQYNSYPLTFGPGTKVDIK(SEQ ID NO:42)
aPSMA heavy-chain: 
EVQLVQSGPEVKKPGATVKISCKTSGYTFTEYTIHWVKQAPGKGLEWIGNINPNNGGTTYNQKFEDK
ATLTVDKSTDTAYMELSSLRSEDTAVYYCAAGWNFDYWGQGTLLTVSSASTKGPSVFPLAPSSKSTS
GGTAALGCLVKDYFPEPVTVSWNSGALTSGVHTFPAVLQSSGLYSLSSVVTVPSSSLGTQTYICNVNH
KPSNTKVDKKVEPKSCDKTHTCPPCPAPPVAGPSVFLFPPKPKDTLMISRTPEVTCVVVDVSHEDPEV
KFNWYVDGVEVHNAKTKPREEQYNSTYRVVSVLTVLHQDWLNGKEYKCKVSNKGLPSSIEKTISKA
KGQPREPQVYTLPPSRDELTKNQVSLTCLVKGFYPSDIAVEWESNGQPENNYKTTPPVLDSDGSFFLY
SKLTVDKSRWQQGNVFSCSVMHEALHNHYTQKSLSLSPGK(SEQ ID NO:43)
aPSMA light-chain:

DIQMTQSPSSLSTSVGDRVTLTCKASQDVGTAVDWYQQKPGQSPKLLIYWASTRHTGIPSRFSGSGSG
TDFTLTISSLQPEDFADYYCQQYNSYPLTFGPGTKVDIKKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLS
SPVTKSFNRGEC(SEQ ID NO:44)

Antibodies include a heavy-chain constant region Fc and/or a light-chain constant region Fc, located at the tail of the antibody, which can bind to receptors on immune cells and mediate various immune effects. The heavy-chain Fc and light-chain Fc can be the same or different. The Fc can be derived from different antibody isotypes, such as IgG (IgG1, IgG2, IgG3, or IgG4), IgM, IgA, IgD, and IgE. In a specific embodiment, the heavy and/or light chain Fc can be modified as needed, for example, amino acid substitutions at glycosylation site N297 to reduce glycosylation, amino acid substitutions that enhance or reduce Fc binding to Fc receptors and/or effector functions (such as antibody-dependent cell-mediated cytotoxicity (ADCC) and/or complement-dependent cytotoxicity (CDC)) (e.g., amino acid substitutions at one or more positions 228, 234, 235, 236, 243, 297, 298, 330, 331, 329, 333, 334, 239, 332, 330, 292, 300, 305, 396, 268, 270, 318, 320, 322, 252, 254, 256 of the Fc [see Liu R et al. Antibodies (Basel) 2020, 9, 64; US5624821; US5648260; CN108350076B; CN106661120B]). In specific embodiments, amino acid mutations can be introduced into the Fc as needed to promote heterodimer pairing between the heavy-chain Fc and light-chain Fc, including but not limited to, for example, using Fc with knob and hole structures respectively, or Fc with charged amino acid substitutions, as long as they promote heterodimer pairing between the heavy-chain Fc and light-chain Fc [see US7695936, US20030078385, US2020040075A1].

Owing to the favorable stability, prolonged half-life, and high affinity for Fc receptors of IgG, which enables efficient binding to Fc receptors, in a preferred embodiment, the antibody targeting surface antigens of therapeutic-related cells comprises an Fc fragment. Preferably, the Fc fragment is selected from Fc fragments of human IgG1, IgG2, IgG3 and IgG4.

A linker peptide is used to connect two molecules to be fused, providing sufficient spatial distance between them to maintain their respective spatial configurations and biological activities. Specifically, a suitable linker can be reasonably selected from existing linkers, or screened based on actual performance. In a preferred embodiment, the linker peptide is a cleavable or non-cleavable linker. The cleavable linker peptide is a self-cleavable, enzymatically cleavable, or chemically cleavable linker peptide. Enzymatic cleavage includes cleavage by endopeptidases or exopeptidases. A linker peptide with better connection efficiency is selected according to the types of amino acids to be linked. In a preferred embodiment, each linker is independently selected from [GGGGS]n (n is selected from 1-6, when n=6, the linker is as shown in SEQ ID NO: 61: GGGGSGGGGSGGGGSGGGGSGGGGSGGGGS), EAAAK (SEQ ID NO: 79), EAAAKEAAAK (SEQ ID NO: 80), GGGGSIPVSLRSGGGGGSG (SEQ ID NO: 65) or GGGGSIPVSLRSGGGSG (SEQ ID NO: 62), GGGGSG (SEQ ID NO: 145), GGGGSGGGGSG (SEQ ID NO: 146), GGGGSGGGGSGGGGSG (SEQ ID NO: 64). More preferably GGGGS (SEQ ID NO: 144), GGGGSG (SEQ ID NO: 145), GGGGSGGGGS (SEQ ID NO: 63), GGGGSGGGGSG (SEQ ID NO: 146), GGGGSGGGGSGGGGSG (SEQ ID NO: 64), GGGGSIPVSLRSGGGGGSG (SEQ ID NO: 65) or GGGGSIPVSLRSGGGSG (SEQ ID NO: 62).

In practical applications, suitable IL-15 and IL-15Rα amino acid sequences can be embedded and fused with different antibodies based on specific effects. In a preferred embodiment, the fusion antibody comprises a heavy-chain and a light-chain having any one of the following sets of amino acid sequences, respectively: 1) SEQ ID NO: 1 and SEQ ID NO: 2; 2) SEQ ID NO: 3 and SEQ ID NO: 4; 3) SEQ ID NO: 1 and SEQ ID NO: 5; 4) SEQ ID NO: 1 and SEQ ID NO: 6; 5) SEQ ID NO: 7 and SEQ ID NO: 8; 6) SEQ ID NO: 9 and SEQ ID NO: 10; 7) SEQ ID NO: 5 and SEQ ID NO: 11; 8) SEQ ID NO: 45 and SEQ ID NO: 46; 9) SEQ ID NO: 57 and SEQ ID NO: 58; 10) SEQ ID NO: 59 and SEQ ID NO: 60; 11) SEQ ID NO: 57 and SEQ ID NO: 60; 12) SEQ ID NO: 67 and SEQ ID NO: 68; 13) SEQ ID NO: 69 and SEQ ID NO: 70; 14) SEQ ID NO: 71 and SEQ ID NO: 72; 15) SEQ ID NO: 73 and SEQ ID NO: 74; 16) SEQ ID NO: 75 and SEQ ID NO: 76; 17) SEQ ID NO: 77 and SEQ ID NO: 78; 18) SEQ ID NO: 82 and SEQ ID NO: 8; 19) SEQ ID NO: 83 and SEQ ID NO: 84; 20) SEQ ID NO: 85 and SEQ ID NO: 84; 21) SEQ ID NO: 83 and SEQ ID NO: 86; 22) SEQ ID NO: 85 and SEQ ID NO: 86; 23) SEQ ID NO: 88 and SEQ ID NO: 89; 24) SEQ ID NO: 90 and SEQ ID NO: 91; 25) SEQ ID NO: 92 and SEQ ID NO: 93; 26) SEQ ID NO: 94 and SEQ ID NO: 95; 27) SEQ ID NO: 96 and SEQ ID NO: 97; 28) SEQ ID NO: 98 and SEQ ID NO: 99; 29) SEQ ID NO: 102 and SEQ ID NO: 103; 30) SEQ ID NO: 106 and SEQ ID NO: 107; 31) SEQ ID NO: 108 and SEQ ID NO: 107; 32) SEQ ID NO: 112 and SEQ ID NO: 113; 33) SEQ ID NO: 112 and SEQ ID NO: 114; 34) SEQ ID NO: 115 and SEQ ID NO: 116; 35) SEQ ID NO: 115 and SEQ ID NO: 117.

Wherein, as required, the Fc is subjected to null mutations that attenuate ADCC/CDC functions(relative to wild-type, the null mutation sites are E233P, L234V, del235L, G236A, A327G, A330S, P331S). In some specific embodiments, the Fc adopts a Knob-into-hole format; the mutation sites of Fc (knob) are S354C, T366W; the mutation sites of Fc (hole) are Y349C, T366S, L368A, Y407V) (Kabat EU numbering).
SEQ ID NO:1 aPDL1 VH-hIL15-CH1-Fc 
EVQLVESGGGLVQPGGSLRLSCAASGFTFSDSWIHWVRQAPGKGLEWVAWISPYGGSTYYADSV
KGRFTISADTSKNTAYLQMNSLRAEDTAVYYCARRHWPGGFDYWGQGTLVTVSSGGGGSGGGG
SGGGGSGNWVNVISDLKKIEDLIQSMHIDATLYTESDVHPSCKVTAMKCFLLELQVISLESGDASI
HDTVENLIILANDSLSSNGNVTESGCKECEELEEKNIKEFLQSFVHIVQMFINTSGGGGSGGGGSA
STKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPPVAGPSVFLFPPKPKDTL
MISRTPEVTCVVVDVSHEDPEVKFNWYVDGVEVHNAKTKPREEQYNSTYRVVSVLTVLHQDWL
NGKEYKCKVSNKGLPSSIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSLS
PGK
SEQ ID NO:2 aPDL1 VL-hIL15RaSushi-CL 
DIQMTQSPSSLSASVGDRVTITCRASQDVSTAVAWYQQKPGKAPKLLIYSASFLYSGVPSRFSGSG
SGTDFTLTISSLQPEDFATYYCQQYLYHPATFGQGTKVEIGGGGSGGGGSGGGGSGITCPPPMSVE
HADIWVKSYSLYSRERYICNSGFKRKAGTSSLTECVLNKATNVAHWTTPSLKCIRGGGGSGGGGS
KRTVAAPSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC
SEQ ID NO:3 aPDL1 VH-hIL15RaSushi-CH1-Fc 
EVQLVESGGGLVQPGGSLRLSCAASGFTFSDSWIHWVRQAPGKGLEWVAWISPYGGSTYYADSV
KGRFTISADTSKNTAYLQMNSLRAEDTAVYYCARRHWPGGFDYWGQGTLVTVSSGGGGSGGGG
SGGGGSGITCPPPMSVEHADIWVKSYSLYSRERYICNSGFKRKAGTSSLTECVLNKATNVAHWTT
PSLKCIRGGGGSGGGGSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTHTCPPCPAP
PVAGPSVFLFPPKPKDTLMISRTPEVTCVVVDVSHEDPEVKFNWYVDGVEVHNAKTKPREEQYN
 
STYRVVSVLTVLHQDWLNGKEYKCKVSNKGLPSSIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSV
MHEALHNHYTQKSLSLSPGK
SEQ ID NO:4 aPDL1 VL-hIL15-CL 
DIQMTQSPSSLSASVGDRVTITCRASQDVSTAVAWYQQKPGKAPKLLIYSASFLYSGVPSRFSGSG
SGTDFTLTISSLQPEDFATYYCQQYLYHPATFGQGTKVEIGGGGSGGGGSGGGGSGNWVNVISDL
KKIEDLIQSMHIDATLYTESDVHPSCKVTAMKCFLLELQVISLESGDASIHDTVENLIILANDSLSSN
GNVTESGCKECEELEEKNIKEFLQSFVHIVQMFINTSGGGGSGGGGSKRTVAAPSVFIFPPSDEQL
KSGTASVVCLLNNFYPREAKVQWKVDNALQSGNSQESVTEQDSKDSTYSLSSTLTLSKADYEKH
KVYACEVTHQGLSSPVTKSFNRGEC
SEQ ID NO:5 aPDL1 VL-MP2GS-hIL15RaSushi-CL 
DIQMTQSPSSLSASVGDRVTITCRASQDVSTAVAWYQQKPGKAPKLLIYSASFLYSGVPSRFSGSG
SGTDFTLTISSLQPEDFATYYCQQYLYHPATFGQGTKVEIGGGGSIPVSLRSGGGGGSGITCPPPMS
VEHADIWVKSYSLYSRERYICNSGFKRKAGTSSLTECVLNKATNVAHWTTPSLKCIRGGGGSGGG
GSKRTVAAPSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC
SEQ ID NO:6 aPDL1 VL-MP2-hIL15RaSushi-CL 
DIQMTQSPSSLSASVGDRVTITCRASQDVSTAVAWYQQKPGKAPKLLIYSASFLYSGVPSRFSGSG
SGTDFTLTISSLQPEDFATYYCQQYLYHPATFGQGTKVEIGGGGSIPVSLRSGGGSGITCPPPMSVE
HADIWVKSYSLYSRERYICNSGFKRKAGTSSLTECVLNKATNVAHWTTPSLKCIRGGGGSGGGGS
KRTVAAPSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC
SEQ ID NO:7 J591 VH-hIL15-CH1-Fc 
EVQLVQSGPEVKKPGATVKISCKTSGYTFTEYTIHWVKQAPGKGLEWIGNINPNNGGTTYNQKFE
DKATLTVDKSTDTAYMELSSLRSEDTAVYYCAAGWNFDYWGQGTLLTVSSGGGGSGGGGSGGG
GSGNWVNVISDLKKIEDLIQSMHIDATLYTESDVHPSCKVTAMKCFLLELQVISLESGDASIHDTV
ENLIILANDSLSSNGNVTESGCKECEELEEKNIKEFLQSFVHIVQMFINTSGGGGSGGGGSASTKG
PSVFPLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPPVAGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKGLPSSIEKTISKAKGQPREPQVYTLPPSRDELTKNQVSLTCLVKGFYPSDIAVEWESN
GQPENNYKTTPPVLDSDGSFFLYSKLTVDKSRWQQGNVFSCSVMHEALHNHYTQKSLSLSPGK
SEQ ID NO:8 J591 VL-hIL15RaSushi-CL 
DIQMTQSPSSLSTSVGDRVTLTCKASQDVGTAVDWYQQKPGQSPKLLIYWASTRHTGIPSRFSGSG
SGTDFTLTISSLQPEDFADYYCQQYNSYPLTFGPGTKVDIKGGGGSGGGGSGGGGSGITCPPPMSV
EHADIWVKSYSLYSRERYICNSGFKRKAGTSSLTECVLNKATNVAHWTTPSLKCIRGGGGSGGGG
SKRTVAAPSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC。
SEQ ID NO:9 aB7H3 VH-hIL15-CH1-Fc 
QVQLQQSGAELVKPGASVKLSCKASGYTFTNYDINWVRQRPEQGLEWIGWIFPGDGSTQYNEKF
KGKATLTTDTSSSTAYMQLSRLTSEDSAVYFCARQTTATWFAYWGQGTLVTVSAGGGGSGGGGS
GGGGSGNWVNVISDLKKIEDLIQSMHIDATLYTESDVHPSCKVTAMKCFLLELQVISLESGDASIH
DTVENLIILANDSLSSNGNVTESGCKECEELEEKNIKEFLQSFVHIVQMFINTSGGGGSGGGGSAS
TKGPSVFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPPVAGPSVFLFPPKPKDTLM
ISRTPEVTCVVVDVSHEDPEVKFNWYVDGVEVHNAKTKPREEQYNSTYRVVSVLTVLHQDWLN
GKEYKCKVSNKGLPSSIEKTISKAKGQPREPQVYTLPPSRDELTKNQVSLTCLVKGFYPSDIAVEW
ESNGQPENNYKTTPPVLDSDGSFFLYSKLTVDKSRWQQGNVFSCSVMHEALHNHYTQKSLSLSP
GK
SEQ ID NO:10 aB7H3 VL-hIL15RaSushi-CL 
DIVMTQSPATLSVTPGDRVSLSCRASQSISDYLHWYQQKSHESPRLLIKYASQSISGIPSRFSGSGSG
SDFTLSINSVEPEDVGVYYCQNGHSFPLTFGAGTKLELKGGGGSGGGGSGGGGSGITCPPPMSVE
HADIWVKSYSLYSRERYICNSGFKRKAGTSSLTECVLNKATNVAHWTTPSLKCIRGGGGSGGGGS
KRTVAAPSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC
SEQ ID NO:11 aPDL1 VH-MP2GS-hIL15-CH1-Fc 
EVQLVESGGGLVQPGGSLRLSCAASGFTFSDSWIHWVRQAPGKGLEWVAWISPYGGSTYYADSV
KGRFTISADTSKNTAYLQMNSLRAEDTAVYYCARRHWPGGFDYWGQGTLVTVSSGGGGSIPVSL
RSGGGSGNWVNVISDLKKIEDLIQSMHIDATLYTESDVHPSCKVTAMKCFLLELQVISLESGDASI
HDTVENLIILANDSLSSNGNVTESGCKECEELEEKNIKEFLQSFVHIVQMFINTSGGGGSGGGGSA
STKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPPVAGPSVFLFPPKPKDTL
MISRTPEVTCVVVDVSHEDPEVKFNWYVDGVEVHNAKTKPREEQYNSTYRVVSVLTVLHQDWL
NGKEYKCKVSNKGLPSSIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSLS
PGK
SEQ ID NO:45 aPDL1 VH-hIL15-CH1-Fc(hole) 
EVQLVESGGGLVQPGGSLRLSCAASGFTFSDSWIHWVRQAPGKGLEWVAWISPYGGSTYYADSV
KGRFTISADTSKNTAYLQMNSLRAEDTAVYYCARRHWPGGFDYWGQGTLVTVSSGGGGSGGGG
SGGGGSGNWVNVISDLKKIEDLIQSMHIDATLYTESDVHPSCKVTAMKCFLLELQVISLESGDASI
HDTVENLIILANDSLSSNGNVTESGCKECEELEEKNIKEFLQSFVHIVQMFINTSGGGGSGGGGSA
STKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPPVAGPSVFLFPPKPKDTL
MISRTPEVTCVVVDVSHEDPEVKFNWYVDGVEVHNAKTKPREEQYNSTYRVVSVLTVLHQDWL
NGKEYKCKVSNKGLPSSIEKTISKAKGQPREPQVCTLPPSRDELTKNQVSLSCAVKGFYPSDIAVE
WESNGQPENNYKTTPPVLDSDGSFFLVSKLTVDKSRWQQGNVFSCSVMHEALHNHYTQKSLSLS
PGK
SEQ ID NO:46 aPDL1 VL-hIL15RaSushi-CL-Fc(knob) 
DIQMTQSPSSLSASVGDRVTITCRASQDVSTAVAWYQQKPGKAPKLLIYSASFLYSGVPSRFSGSG
SGTDFTLTISSLQPEDFATYYCQQYLYHPATFGQGTKVEIGGGGSGGGGSGGGGSGITCPPPMSVE
HADIWVKSYSLYSRERYICNSGFKRKAGTSSLTECVLNKATNVAHWTTPSLKCIRGGGGSGGGGS
KRTVAAPSVFIFPPSDEQLKSGTASVVCLLNNFYPREAKVQWKVDNALQSGNSQESVTEQDSKDS
 
TYSLSSTLTLSKADYEKHKVYACEVTHQGLSSPVTKSFNRGECDKTHTCPPCPAPPVAGPSVFLFP
PKPKDTLMISRTPEVTCVVVDVSHEDPEVKFNWYVDGVEVHNAKTKPREEQYNSTYRVVSVLT
VLHQDWLNGKEYKCKVSNKGLPSSIEKTISKAKGQPREPQVYTLPPCRDELTKNQVSLWCLVKG
FYPSDIAVEWESNGQPENNYKTTPPVLDSDGSFFLYSKLTVDKSRWQQGNVFSCSVMHEALHNH
YTQKSLSLSPGK
SEQ ID NO:47 hIL15-aPDL1-VH-CH1-Fc 
NWVNVISDLKKIEDLIQSMHIDATLYTESDVHPSCKVTAMKCFLLELQVISLESGDASIHDTVENLI
ILANDSLSSNGNVTESGCKECEELEEKNIKEFLQSFVHIVQMFINTSGGGGSGGGGSGGGGSGEV
QLVESGGGLVQPGGSLRLSCAASGFTFSDSWIHWVRQAPGKGLEWVAWISPYGGSTYYADSVKG
RFTISADTSKNTAYLQMNSLRAEDTAVYYCARRHWPGGFDYWGQGTLVTVSSASTKGPSVFPLA
PSSKSTSGGTAALGCLVKDYFPEPVTVSWNSGALTSGVHTFPAVLQSSGLYSLSSVVTVPSSSLGT
QTYICNVNHKPSNTKVDKKVEPKSCDKTHTCPPCPAPPVAGPSVFLFPPKPKDTLMISRTPEVTCV
VVDVSHEDPEVKFNWYVDGVEVHNAKTKPREEQYNSTYRVVSVLTVLHQDWLNGKEYKCKVS
NKGLPSSIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK
SEQ ID NO:48 hIL15RaSushi-aPDL1-VL-CH1 
ITCPPPMSVEHADIWVKSYSLYSRERYICNSGFKRKAGTSSLTECVLNKATNVAHWTTPSLKCIRG
GGGSGGGGSGGGGSGDIQMTQSPSSLSASVGDRVTITCRASQDVSTAVAWYQQKPGKAPKLLIYS
ASFLYSGVPSRFSGSGSGTDFTLTISSLQPEDFATYYCQQYLYHPATFGQGTKVEIKRTVAAPSVFIF
PPSDEQLKSGTASVVCLLNNFYPREAKVQWKVDNALQSGNSQESVTEQDSKDSTYSLSSTLTLSK
ADYEKHKVYACEVTHQGLSSPVTKSFNRGEC
SEQ ID NO:49 hIL15-aB7H3-VH-CH1-Fc 
NWVNVISDLKKIEDLIQSMHIDATLYTESDVHPSCKVTAMKCFLLELQVISLESGDASIHDTVENLI
ILANDSLSSNGNVTESGCKECEELEEKNIKEFLQSFVHIVQMFINTSGGGGSGGGGSGGGGSGQV
QLQQSGAELVKPGASVKLSCKASGYTFTNYDINWVRQRPEQGLEWIGWIFPGDGSTQYNEKFKG
KATLTTDTSSSTAYMQLSRLTSEDSAVYFCARQTTATWFAYWGQGTLVTVSAASTKGPSVFPLAPS
SKSTSGGTAALGCLVKDYFPEPVTVSWNSGALTSGVHTFPAVLQSSGLYSLSSVVTVPSSSLGTQT
YICNVNHKPSNTKVDKKVEPKSCDKTHTCPPCPAPPVAGPSVFLFPPKPKDTLMISRTPEVTCVVV
DVSHEDPEVKFNWYVDGVEVHNAKTKPREEQYNSTYRVVSVLTVLHQDWLNGKEYKCKVSNK
GLPSSIEKTISKAKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPGK
SEQ ID NO:50 hIL15RaSushi-aB7H3-VL-CL 
ITCPPPMSVEHADIWVKSYSLYSRERYICNSGFKRKAGTSSLTECVLNKATNVAHWTTPSLKCIRG
GGGSGGGGSGGGGSGDIVMTQSPATLSVTPGDRVSLSCRASQSISDYLHWYQQKSHESPRLLIKY
ASQSISGIPSRFSGSGSGSDFTLSINSVEPEDVGVYYCQNGHSFPLTFGAGTKLELKKRTVAAPSVFI
FPPSDEQLKSGTASVVCLLNNFYPREAKVQWKVDNALQSGNSQESVTEQDSKDSTYSLSSTLTLS
KADYEKHKVYACEVTHQGLSSPVTKSFNRGEC
SEQ ID NO:51 hIL15-aPSMA-VH-CH1-Fc 
NWVNVISDLKKIEDLIQSMHIDATLYTESDVHPSCKVTAMKCFLLELQVISLESGDASIHDTVENLI
ILANDSLSSNGNVTESGCKECEELEEKNIKEFLQSFVHIVQMFINTSGGGGSGGGGSGGGGSGEV
QLVQSGPEVKKPGATVKISCKTSGYTFTEYTIHWVKQAPGKGLEWIGNINPNNGGTTYNQKFED
KATLTVDKSTDTAYMELSSLRSEDTAVYYCAAGWNFDYWGQGTLLTVSSASTKGPSVFPLAPSSK
 
STSGGTAALGCLVKDYFPEPVTVSWNSGALTSGVHTFPAVLQSSGLYSLSSVVTVPSSSLGTQTYI
CNVNHKPSNTKVDKKVEPKSCDKTHTCPPCPAPPVAGPSVFLFPPKPKDTLMISRTPEVTCVVVD
VSHEDPEVKFNWYVDGVEVHNAKTKPREEQYNSTYRVVSVLTVLHQDWLNGKEYKCKVSNKG
LPSSIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGK
SEQ ID NO:52 hIL15RaSushi-aPSMA-VL-CL 
ITCPPPMSVEHADIWVKSYSLYSRERYICNSGFKRKAGTSSLTECVLNKATNVAHWTTPSLKCIRG
GGGSGGGGSGGGGSGDIQMTQSPSSLSTSVGDRVTLTCKASQDVGTAVDWYQQKPGQSPKLLIY
WASTRHTGIPSRFSGSGSGTDFTLTISSLQPEDFADYYCQQYNSYPLTFGPGTKVDIKKRTVAAPSV
FIFPPSDEQLKSGTASVVCLLNNFYPREAKVQWKVDNALQSGNSQESVTEQDSKDSTYSLSSTLT
LSKADYEKHKVYACEVTHQGLSSPVTKSFNRGEC
SEQ ID NO:53 hIL15-aPDL1-VH-CH1-Fc(hole) 
NWVNVISDLKKIEDLIQSMHIDATLYTESDVHPSCKVTAMKCFLLELQVISLESGDASIHDTVENLI
ILANDSLSSNGNVTESGCKECEELEEKNIKEFLQSFVHIVQMFINTSGGGGSGGGGSGGGGSGEV
QLVESGGGLVQPGGSLRLSCAASGFTFSDSWIHWVRQAPGKGLEWVAWISPYGGSTYYADSVKG
RFTISADTSKNTAYLQMNSLRAEDTAVYYCARRHWPGGFDYWGQGTLVTVSSASTKGPSVFPLA
PSSKSTSGGTAALGCLVKDYFPEPVTVSWNSGALTSGVHTFPAVLQSSGLYSLSSVVTVPSSSLGT
QTYICNVNHKPSNTKVDKKVEPKSCDKTHTCPPCPAPPVAGPSVFLFPPKPKDTLMISRTPEVTCV
VVDVSHEDPEVKFNWYVDGVEVHNAKTKPREEQYNSTYRVVSVLTVLHQDWLNGKEYKCKVS
NKGLPSSIEKTISKAKGQPREPQVCTLPPSRDELTKNQVSLSCAVKGFYPSDIAVEWESNGQPENN
YKTTPPVLDSDGSFFLVSKLTVDKSRWQQGNVFSCSVMHEALHNHYTQKSLSLSPGK
SEQ ID NO:54 hIL15RaSushi-aPDL1-VL-CL-Fc(knob) 
ITCPPPMSVEHADIWVKSYSLYSRERYICNSGFKRKAGTSSLTECVLNKATNVAHWTTPSLKCIRG
GGGSGGGGSGGGGSGDIQMTQSPSSLSASVGDRVTITCRASQDVSTAVAWYQQKPGKAPKLLIYS
ASFLYSGVPSRFSGSGSGTDFTLTISSLQPEDFATYYCQQYLYHPATFGQGTKVEIKRTVAAPSVFIF
PPSDEQLKSGTASVVCLLNNFYPREAKVQWKVDNALQSGNSQESVTEQDSKDSTYSLSSTLTLSK
ADYEKHKVYACEVTHQGLSSPVTKSFNRGECDKTHTCPPCPAPPVAGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKGLPSSIEKTISKAKGQPREPQVYTLPPCRDELTKNQVSLWCLVKGFYPSDIAVEWESN
GQPENNYKTTPPVLDSDGSFFLYSKLTVDKSRWQQGNVFSCSVMHEALHNHYTQKSLSLSPGK
SEQ ID NO:55 hIL15-aEGFR-VH-CH1-Fc 
NWVNVISDLKKIEDLIQSMHIDATLYTESDVHPSCKVTAMKCFLLELQVISLESGDASIHDTVENLI
ILANDSLSSNGNVTESGCKECEELEEKNIKEFLQSFVHIVQMFINTSGGGGSGGGGSGGGGSGQV
QLQESGPGLVKPSETLSLTCTVSGGSVSSGDYYWTWIRQSPGKGLEWIGHIYYSGNTNYNPSLKS
RLTISIDTSKTQFSLKLSSVTAADTAIYYCVRDRVTGAFDIWGQGTMVTVSSASTKGPSVFPLAPSS
KSTSGGTAALGCLVKDYFPEPVTVSWNSGALTSGVHTFPAVLQSSGLYSLSSVVTVPSSSLGTQTY
ICNVNHKPSNTKVDKKVEPKSCDKTHTCPPCPAPPVAGPSVFLFPPKPKDTLMISRTPEVTCVVVD
VSHEDPEVKFNWYVDGVEVHNAKTKPREEQYNSTYRVVSVLTVLHQDWLNGKEYKCKVSNKG
LPSSIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGK
SEQ ID NO:56 hIL15RaSushi-aEGFR-VL-CL 
ITCPPPMSVEHADIWVKSYSLYSRERYICNSGFKRKAGTSSLTECVLNKATNVAHWTTPSLKCIRG
GGGSGGGGSGGGGSGDIQMTQSPSSLSASVGDRVTITCQASQDISNYLNWYQQKPGKAPKLLIY
DASNLETGVPSRFSGSGSGTDFTFTISSLQPEDIATYFCQHFDHLPLAFGGGTKVEIKRTVAAPSVFI
FPPSDEQLKSGTASVVCLLNNFYPREAKVQWKVDNALQSGNSQESVTEQDSKDSTYSLSSTLTLS
KADYEKHKVYACEVTHQGLSSPVTKSFNRGEC
SEQ ID NO:57 aPDL1 VH-MP2GS-hIL15RaSushi-CH1-Fc(hole) 
EVQLVESGGGLVQPGGSLRLSCAASGFTFSDSWIHWVRQAPGKGLEWVAWISPYGGSTYYADSV
KGRFTISADTSKNTAYLQMNSLRAEDTAVYYCARRHWPGGFDYWGQGTLVTVSSGGGGSIPVSL
RSGGGGGSGITCPPPMSVEHADIWVKSYSLYSRERYICNSGFKRKAGTSSLTECVLNKATNVAHW
TTPSLKCIRGGGGSEPKSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKVEPKSCDKTHTCPPCP
APPVAGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKGLPSSIEKTISKAKGQPREPQVCTLPPSRDELTK
NQVSLSCAVKGFYPSDIAVEWESNGQPENNYKTTPPVLDSDGSFFLVSKLTVDKSRWQQGNVFSC
SVMHEALHNHYTQKSLSLSPGK
SEQ ID NO:58 aPDL1 VL-hIL15-CL-Fc(knob) 
DIQMTQSPSSLSASVGDRVTITCRASQDVSTAVAWYQQKPGKAPKLLIYSASFLYSGVPSRFSGSG
SGTDFTLTISSLQPEDFATYYCQQYLYHPATFGQGTKVEIGGGGSGGGGSGGGGSGNWVNVISDL
KKIEDLIQSMHIDATLYTESDVHPSCKVTAMKCFLLELQVISLESGDASIHDTVENLIILANDSLSSN
GNVTESGCKECEELEEKNIKEFLQSFVHIVQMFINTSGGGGSGGGGSKRTVAAPSVFIFPPSDEQL
KSGTASVVCLLNNFYPREAKVQWKVDNALQSGNSQESVTEQDSKDSTYSLSSTLTLSKADYEKH
KVYACEVTHQGLSSPVTKSFNRGECDKTHTCPPCPAPPVAGPSVFLFPPKPKDTLMISRTPEVTCV
VVDVSHEDPEVKFNWYVDGVEVHNAKTKPREEQYNSTYRVVSVLTVLHQDWLNGKEYKCKVS
NKGLPSSIEKTISKAKGQPREPQVYTLPPCRDELTKNQVSLWCLVKGFYPSDIAVEWESNGQPENN
YKTTPPVLDSDGSFFLYSKLTVDKSRWQQGNVFSCSVMHEALHNHYTQKSLSLSPGK
SEQ ID NO:59 aPDL1 VH-hIL15RaSushi-CH1-Fc(hole) 
EVQLVESGGGLVQPGGSLRLSCAASGFTFSDSWIHWVRQAPGKGLEWVAWISPYGGSTYYADSV
KGRFTISADTSKNTAYLQMNSLRAEDTAVYYCARRHWPGGFDYWGQGTLVTVSSGGGGSGGGG
SGGGGSGITCPPPMSVEHADIWVKSYSLYSRERYICNSGFKRKAGTSSLTECVLNKATNVAHWTT
PSLKCIRGGGGSGGGGSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTHTCPPCPAP
PVAGPSVFLFPPKPKDTLMISRTPEVTCVVVDVSHEDPEVKFNWYVDGVEVHNAKTKPREEQYN
STYRVVSVLTVLHQDWLNGKEYKCKVSNKGLPSSIEKTISKAKGQPREPQVCTLPPSRDELTKNQ
VSLSCAVKGFYPSDIAVEWESNGQPENNYKTTPPVLDSDGSFFLVSKLTVDKSRWQQGNVFSCSV
MHEALHNHYTQKSLSLSPGK
SEQ ID NO:60 aPDL1 VL-MP2GS-hIL15-CL-Fc(knob) 
DIQMTQSPSSLSASVGDRVTITCRASQDVSTAVAWYQQKPGKAPKLLIYSASFLYSGVPSRFSGSG
SGTDFTLTISSLQPEDFATYYCQQYLYHPATFGQGTKVEIGGGGSIPVSLRSGGGGGSGNWVNVIS
DLKKIEDLIQSMHIDATLYTESDVHPSCKVTAMKCFLLELQVISLESGDASIHDTVENLIILANDSL
SSNGNVTESGCKECEELEEKNIKEFLQSFVHIVQMFINTSGGGGSGGGGSKRTVAAPSVFIFPPSDE
QLKSGTASVVCLLNNFYPREAKVQWKVDNALQSGNSQESVTEQDSKDSTYSLSSTLTLSKADYE
KHKVYACEVTHQGLSSPVTKSFNRGECDKTHTCPPCPAPPVAGPSVFLFPPKPKDTLMISRTPEVT
CVVVDVSHEDPEVKFNWYVDGVEVHNAKTKPREEQYNSTYRVVSVLTVLHQDWLNGKEYKCK
 
VSNKGLPSSIEKTISKAKGQPREPQVYTLPPCRDELTKNQVSLWCLVKGFYPSDIAVEWESNGQPE
NNYKTTPPVLDSDGSFFLYSKLTVDKSRWQQGNVFSCSVMHEALHNHYTQKSLSLSPGK
SEQ ID NO:67 aPSMA VH-10GS-hIL15-10GS-CH1-Fc 
EVQLVQSGPEVKKPGATVKISCKTSGYTFTEYTIHWVKQAPGKGLEWIGNINPNNGGTTYNQKFE
DKATLTVDKSTDTAYMELSSLRSEDTAVYYCAAGWNFDYWGQGTLLTVSSGGGGSGGGGSNWV
NVISDLKKIEDLIQSMHIDATLYTESDVHPSCKVTAMKCFLLELQVISLESGDASIHDTVENLIILAN
NSLSSNGNVTESGCKECEELEEKNIKEFLQSFVHIVQMFINTSGGGGSGGGGSASTKGPSVFPLAP
SSKSTSGGTAALGCLVKDYFPEPVTVSWNSGALTSGVHTFPAVLQSSGLYSLSSVVTVPSSSLGTQ
TYICNVNHKPSNTKVDKKVEPKSCDKTHTCPPCPAPPVAGPSVFLFPPKPKDTLMISRTPEVTCVV
VDVSHEDPEVKFNWYVDGVEVHNAKTKPREEQYNSTYRVVSVLTVLHQDWLNGKEYKCKVSN
KGLPSSIEKTISKAKGQPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSLSLSPGK
SEQ ID NO:68 aPSMA VL-10GS-hIL15RaSushi-10GS-CL 
DIQMTQSPSSLSTSVGDRVTLTCKASQDVGTAVDWYQQKPGQSPKLLIYWASTRHTGIPSRFSGSG
SGTDFTLTISSLQPEDFADYYCQQYNSYPLTFGPGTKVDIKGGGGSGGGGSITCPPPMSVEHADIW
VKSYSLYSRERYICNSGFKRKAGTSSLTECVLNKATNVAHWTTPSLKCIRGGGGSGGGGSKRTVA
APSVFIFPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC
SEQ ID NO:69: aPSMA VH-5GS-hIL15-10GS-CH1-Fc 
EVQLVQSGPEVKKPGATVKISCKTSGYTFTEYTIHWVKQAPGKGLEWIGNINPNNGGTTYNQKFE
DKATLTVDKSTDTAYMELSSLRSEDTAVYYCAAGWNFDYWGQGTLLTVSSGGGGSNWVNVISD
LKKIEDLIQSMHIDATLYTESDVHPSCKVTAMKCFLLELQVISLESGDASIHDTVENLIILANNSLSS
NGNVTESGCKECEELEEKNIKEFLQSFVHIVQMFINTSGGGGSGGGGSASTKGPSVFPLAPSSKST
SGGTAALGCLVKDYFPEPVTVSWNSGALTSGVHTFPAVLQSSGLYSLSSVVTVPSSSLGTQTYICN
VNHKPSNTKVDKKVEPKSCDKTHTCPPCPAPPVAGPSVFLFPPKPKDTLMISRTPEVTCVVVDVS
HEDPEVKFNWYVDGVEVHNAKTKPREEQYNSTYRVVSVLTVLHQDWLNGKEYKCKVSNKGLP
SSIEKTISKAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK
SEQ ID NO:70: aPSMA VL-5GS-hIL15RaSushi-10GS-CL 
DIQMTQSPSSLSTSVGDRVTLTCKASQDVGTAVDWYQQKPGQSPKLLIYWASTRHTGIPSRFSGSG
SGTDFTLTISSLQPEDFADYYCQQYNSYPLTFGPGTKVDIKGGGGSITCPPPMSVEHADIWVKSYS
LYSRERYICNSGFKRKAGTSSLTECVLNKATNVAHWTTPSLKCIRGGGGSGGGGSKRTVAAPSVFI
FPPSDEQLKSGTASVVCLLNNFYPREAKVQWKVDNALQSGNSQESVTEQDSKDSTYSLSSTLTLS
KADYEKHKVYACEVTHQGLSSPVTKSFNRGEC
SEQ ID NO:71: aPSMA VH-5GS-hIL15-5GS-CH1-Fc 
EVQLVQSGPEVKKPGATVKISCKTSGYTFTEYTIHWVKQAPGKGLEWIGNINPNNGGTTYNQKFE
DKATLTVDKSTDTAYMELSSLRSEDTAVYYCAAGWNFDYWGQGTLLTVSSGGGGSNWVNVISD
LKKIEDLIQSMHIDATLYTESDVHPSCKVTAMKCFLLELQVISLESGDASIHDTVENLIILANNSLSS
NGNVTESGCKECEELEEKNIKEFLQSFVHIVQMFINTSGGGGSASTKGPSVFPLAPSSKSTSGGTA
ALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPPVAGPSVFLFPPKPKDTLMISRTPEVTCVVVDVSHEDPEV
KFNWYVDGVEVHNAKTKPREEQYNSTYRVVSVLTVLHQDWLNGKEYKCKVSNKGLPSSIEKTI
 
SKAKGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK
SEQ ID NO:72: aPSMA VL-5GS-hIL15RaSushi-5GS-CL 
DIQMTQSPSSLSTSVGDRVTLTCKASQDVGTAVDWYQQKPGQSPKLLIYWASTRHTGIPSRFSGSG
SGTDFTLTISSLQPEDFADYYCQQYNSYPLTFGPGTKVDIKGGGGSITCPPPMSVEHADIWVKSYS
LYSRERYICNSGFKRKAGTSSLTECVLNKATNVAHWTTPSLKCIRGGGGSKRTVAAPSVFIFPPSDE
QLKSGTASVVCLLNNFYPREAKVQWKVDNALQSGNSQESVTEQDSKDSTYSLSSTLTLSKADYE
KHKVYACEVTHQGLSSPVTKSFNRGEC
SEQ ID NO:73:aPSMA VH-(EAAAK)2-hIL15-(EAAAK)2-CH1-Fc 
EVQLVQSGPEVKKPGATVKISCKTSGYTFTEYTIHWVKQAPGKGLEWIGNINPNNGGTTYNQKFE
DKATLTVDKSTDTAYMELSSLRSEDTAVYYCAAGWNFDYWGQGTLLTVSSEAAAKEAAAKNW
VNVISDLKKIEDLIQSMHIDATLYTESDVHPSCKVTAMKCFLLELQVISLESGDASIHDTVENLIILA
NNSLSSNGNVTESGCKECEELEEKNIKEFLQSFVHIVQMFINTSEAAAKEAAAKASTKGPSVFPLA
PSSKSTSGGTAALGCLVKDYFPEPVTVSWNSGALTSGVHTFPAVLQSSGLYSLSSVVTVPSSSLGT
QTYICNVNHKPSNTKVDKKVEPKSCDKTHTCPPCPAPPVAGPSVFLFPPKPKDTLMISRTPEVTCV
VVDVSHEDPEVKFNWYVDGVEVHNAKTKPREEQYNSTYRVVSVLTVLHQDWLNGKEYKCKVS
NKGLPSSIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK
SEQ ID NO:74 aPSMA VL-(EAAAK)2-hIL15RaSushi- (EAAAK)2-CL 
DIQMTQSPSSLSTSVGDRVTLTCKASQDVGTAVDWYQQKPGQSPKLLIYWASTRHTGIPSRFSGSG
SGTDFTLTISSLQPEDFADYYCQQYNSYPLTFGPGTKVDIKEAAAKEAAAKITCPPPMSVEHADIW
VKSYSLYSRERYICNSGFKRKAGTSSLTECVLNKATNVAHWTTPSLKCIREAAAKEAAAKKRTVA
APSVFIFPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC
SEQ ID NO:75 aPSMA VH-EAAAK-hIL15-(EAAAK)2-CH1-Fc 
EVQLVQSGPEVKKPGATVKISCKTSGYTFTEYTIHWVKQAPGKGLEWIGNINPNNGGTTYNQKFE
DKATLTVDKSTDTAYMELSSLRSEDTAVYYCAAGWNFDYWGQGTLLTVSSEAAAKNWVNVISD
LKKIEDLIQSMHIDATLYTESDVHPSCKVTAMKCFLLELQVISLESGDASIHDTVENLIILANNSLSS
NGNVTESGCKECEELEEKNIKEFLQSFVHIVQMFINTSEAAAKEAAAKASTKGPSVFPLAPSSKST
SGGTAALGCLVKDYFPEPVTVSWNSGALTSGVHTFPAVLQSSGLYSLSSVVTVPSSSLGTQTYICN
VNHKPSNTKVDKKVEPKSCDKTHTCPPCPAPPVAGPSVFLFPPKPKDTLMISRTPEVTCVVVDVS
HEDPEVKFNWYVDGVEVHNAKTKPREEQYNSTYRVVSVLTVLHQDWLNGKEYKCKVSNKGLP
SSIEKTISKAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK
SEQ ID NO:76 aPSMA VL-EAAAK-hIL15RaSushi- (EAAAK)2-CL 
DIQMTQSPSSLSTSVGDRVTLTCKASQDVGTAVDWYQQKPGQSPKLLIYWASTRHTGIPSRFSGSG
SGTDFTLTISSLQPEDFADYYCQQYNSYPLTFGPGTKVDIKEAAAKITCPPPMSVEHADIWVKSYS
LYSRERYICNSGFKRKAGTSSLTECVLNKATNVAHWTTPSLKCIREAAAKEAAAKKRTVAAPSVFI
FPPSDEQLKSGTASVVCLLNNFYPREAKVQWKVDNALQSGNSQESVTEQDSKDSTYSLSSTLTLS
KADYEKHKVYACEVTHQGLSSPVTKSFNRGEC。
SEQ ID NO:77 aPSMA VH-EAAAK-hIL15-EAAAK-CH1-Fc 
EVQLVQSGPEVKKPGATVKISCKTSGYTFTEYTIHWVKQAPGKGLEWIGNINPNNGGTTYNQKFE
DKATLTVDKSTDTAYMELSSLRSEDTAVYYCAAGWNFDYWGQGTLLTVSSEAAAKNWVNVISD
LKKIEDLIQSMHIDATLYTESDVHPSCKVTAMKCFLLELQVISLESGDASIHDTVENLIILANNSLSS
NGNVTESGCKECEELEEKNIKEFLQSFVHIVQMFINTSEAAAKASTKGPSVFPLAPSSKSTSGGTA
ALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPPVAGPSVFLFPPKPKDTLMISRTPEVTCVVVDVSHEDPEV
KFNWYVDGVEVHNAKTKPREEQYNSTYRVVSVLTVLHQDWLNGKEYKCKVSNKGLPSSIEKTI
SKAKGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK
SEQ ID NO:78 aPSMA VL-EAAAK-hIL15RaSushi-EAAAK-CL 
DIQMTQSPSSLSTSVGDRVTLTCKASQDVGTAVDWYQQKPGQSPKLLIYWASTRHTGIPSRFSGSG
SGTDFTLTISSLQPEDFADYYCQQYNSYPLTFGPGTKVDIKEAAAKITCPPPMSVEHADIWVKSYS
LYSRERYICNSGFKRKAGTSSLTECVLNKATNVAHWTTPSLKCIREAAAKKRTVAAPSVFIFPPSDE
QLKSGTASVVCLLNNFYPREAKVQWKVDNALQSGNSQESVTEQDSKDSTYSLSSTLTLSKADYE
KHKVYACEVTHQGLSSPVTKSFNRGEC
SEQ ID NO:82 aPSMA VH-WT hIL15-CH1-Fc(null) 
EVQLVQSGPEVKKPGATVKISCKTSGYTFTEYTIHWVKQAPGKGLEWIGNINPNNGGTTYNQKFE
DKATLTVDKSTDTAYMELSSLRSEDTAVYYCAAGWNFDYWGQGTLLTVSSGGGGSGGGGSGGG
GSGNWVNVISDLKKIEDLIQSMHIDATLYTESDVHPSCKVTAMKCFLLELQVISLESGDASIHDTV
ENLIILANNSLSSNGNVTESGCKECEELEEKNIKEFLQSFVHIVQMFINTSGGGGSGGGGSASTKG
PSVFPLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPPVAGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKGLPSSIEKTISKAKGQPREPQVYTLPPSRDELTKNQVSLTCLVKGFYPSDIAVEWESN
GQPENNYKTTPPVLDSDGSFFLYSKLTVDKSRWQQGNVFSCSVMHEALHNHYTQKSLSLSPGK
SEQ ID NO:83 aB7H3-2 VH-IL15-CH1-Fc(hIgG1, wt) 
EVQLVQSGAEVKKPGASVKVSCKASGYTFTNYDINWVRQAPGQGLEWIGWIFPGDGSTQYNEK
FKGRATLTTDTSTSTAYMELRSLRSDDTAVYFCARQTTATWFAYWGQGTLVTVSSGGGGSGGGG
SGGGGSGNWVNVISDLKKIEDLIQSMHIDATLYTESDVHPSCKVTAMKCFLLELQVISLESGDASI
HDTVENLIILANDSLSSNGNVTESGCKECEELEEKNIKEFLQSFVHIVQMFINTSGGGGSGGGGSA
STKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRVVSVLTVLHQDW
LNGKEYKCKVSNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNHYTQKSLSL
SPGK
SEQ ID NO:84 aB7H3-2 VL-hIL15RaSUSHI-CL 
DIVMTQSPLSLPVTPGEPASISCRASQSISDYLHWYLQKPGESPRLLIKYASQSISGIPDRFSGSGSG
SDFTLKISRVEAEDVGVYYCQNGHSFPLTFGAGTKLEIKGGGGSGGGGSGGGGSGITCPPPMSVE
HADIWVKSYSLYSRERYICNSGFKRKAGTSSLTECVLNKATNVAHWTTPSLKCIRGGGGSGGGGS
KRTVAAPSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC
SEQ ID NO:85 aB7H3-2 VH-IL15WT-CH1-Fc(hIgG1, wt) 
EVQLVQSGAEVKKPGASVKVSCKASGYTFTNYDINWVRQAPGQGLEWIGWIFPGDGSTQYNEK
FKGRATLTTDTSTSTAYMELRSLRSDDTAVYFCARQTTATWFAYWGQGTLVTVSSGGGGSGGGG
SGGGGSGNWVNVISDLKKIEDLIQSMHIDATLYTESDVHPSCKVTAMKCFLLELQVISLESGDASI
HDTVENLIILANNSLSSNGNVTESGCKECEELEEKNIKEFLQSFVHIVQMFINTSGGGGSGGGGSA
STKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRVVSVLTVLHQDW
LNGKEYKCKVSNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNHYTQKSLSL
SPGK
SEQ ID NO:86 aB7H3-2-LC 
DIVMTQSPLSLPVTPGEPASISCRASQSISDYLHWYLQKPGESPRLLIKYASQSISGIPDRFSGSGSG
SDFTLKISRVEAEDVGVYYCQNGHSFPLTFGAGTKLEIKRTVAAPSVFIFPPSDEQLKSGTASVVCL
LNNFYPREAKVQWKVDNALQSGNSQESVTEQDSKDSTYSLSSTLTLSKADYEKHKVYACEVTH
QGLSSPVTKSFNRGEC
SEQ ID NO:87 aB7H3-2-HC(hIgG1, wt) 
EVQLVQSGAEVKKPGASVKVSCKASGYTFTNYDINWVRQAPGQGLEWIGWIFPGDGSTQYNEK
FKGRATLTTDTSTSTAYMELRSLRSDDTAVYFCARQTTATWFAYWGQGTLVTVSSASTKGPSVFP
LAPSSKSTSGGTAALGCLVKDYFPEPVTVSWNSGALTSGVHTFPAVLQSSGLYSLSSVVTVPSSSL
GTQTYICNVNHKPSNTKVDKKVEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSLSPGK
SEQ ID NO:88 aPD1-2-16GS-IL15RaSUSHI-10GS-LC 
EIVLTQSPATLSLSPGERATLSCRASKGVSTSGYSYLHWYQQKPGQAPRLLIYLASYLESGVPARFS
GSGSGTDFTLTISSLEPEDFAVYYCQHSRDLPLTFGGGTKVEIKGGGGSGGGGSGGGGSGITCPPP
MSVEHADIWVKSYSLYSRERYICNSGFKRKAGTSSLTECVLNKATNVAHWTTPSLKCIRGGGGSG
GGGSRTVAAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
SEQ ID NO:89 aPD1-2-16GS-IL15WT-10GS-HC-IgG4 
QVQLVQSGVEVKKPGASVKVSCKASGYTFTNYYMYWVRQAPGQGLEWMGGINPSNGGTNFNE
KFKNRVTLTTDSSTTTAYMELKSLQFDDTAVYYCARRDYRFDMGFDYWGQGTTVTVSSGGGGS
GGGGSGGGGSGNWVNVISDLKKIEDLIQSMHIDATLYTESDVHPSCKVTAMKCFLLELQVISLES
GDASIHDTVENLIILANNSLSSNGNVTESGCKECEELEEKNIKEFLQSFVHIVQMFINTSGGGGSGG
GGSASTKGPSVFPLAPCSRSTSESTAALGCLVKDYFPEPVTVSWNSGALTSGVHTFPAVLQSSGLY
SLSSVVTVPSSSLGTKTYTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQFNSTYRVVSVLTVLHQD
WLNGKEYKCKVSNKGLPSSIEKTISKAKGQPREPQVYTLPPSQEEMTKNQVSLTCLVKGFYPSDI
AVEWESNGQPENNYKTTPPVLDSDGSFFLYSRLTVDKSRWQEGNVFSCSVMHEALHNHYTQKSL
SLSLGK
SEQ ID NO:90 aPD1-2-11GS-IL15RaSUSHI-10GS-LC 
EIVLTQSPATLSLSPGERATLSCRASKGVSTSGYSYLHWYQQKPGQAPRLLIYLASYLESGVPARFS
GSGSGTDFTLTISSLEPEDFAVYYCQHSRDLPLTFGGGTKVEIKGGGGSGGGGSGITCPPPMSVEH
ADIWVKSYSLYSRERYICNSGFKRKAGTSSLTECVLNKATNVAHWTTPSLKCIRGGGGSGGGGSR
TVAAPSVFIFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC
SEQ ID NO:91 aPD1-2-11GS-IL15WT-10GS-HC-IgG4 
QVQLVQSGVEVKKPGASVKVSCKASGYTFTNYYMYWVRQAPGQGLEWMGGINPSNGGTNFNE
KFKNRVTLTTDSSTTTAYMELKSLQFDDTAVYYCARRDYRFDMGFDYWGQGTTVTVSSGGGGS
GGGGSGNWVNVISDLKKIEDLIQSMHIDATLYTESDVHPSCKVTAMKCFLLELQVISLESGDASIH
DTVENLIILANNSLSSNGNVTESGCKECEELEEKNIKEFLQSFVHIVQMFINTSGGGGSGGGGSAS
TKGPSVFPLAPCSRSTSESTAALGCLVKDYFPEPVTVSWNSGALTSGVHTFPAVLQSSGLYSLSSV
VTVPSSSLGTKTYTCNVDHKPSNTKVDKRVESKYGPPCPPCPAPEFLGGPSVFLFPPKPKDTLMIS
RTPEVTCVVVDVSQEDPEVQFNWYVDGVEVHNAKTKPREEQFNSTYRVVSVLTVLHQDWLNG
KEYKCKVSNKGLPSSIEKTISKAKGQPREPQVYTLPPSQEEMTKNQVSLTCLVKGFYPSDIAVEWE
SNGQPENNYKTTPPVLDSDGSFFLYSRLTVDKSRWQEGNVFSCSVMHEALHNHYTQKSLSLSLG
K
SEQ ID NO:92 aPD1-2-6GS-IL15RaSUSHI-5GS-LC 
EIVLTQSPATLSLSPGERATLSCRASKGVSTSGYSYLHWYQQKPGQAPRLLIYLASYLESGVPARFS
GSGSGTDFTLTISSLEPEDFAVYYCQHSRDLPLTFGGGTKVEIKGGGGSGITCPPPMSVEHADIWV
KSYSLYSRERYICNSGFKRKAGTSSLTECVLNKATNVAHWTTPSLKCIRGGGGSRTVAAPSVFIFPP
SDEQLKSGTASVVCLLNNFYPREAKVQWKVDNALQSGNSQESVTEQDSKDSTYSLSSTLTLSKA
DYEKHKVYACEVTHQGLSSPVTKSFNRGEC
SEQ ID NO:93 aPD1-2-6GS-IL15WT-5GS-HC-IgG4 
QVQLVQSGVEVKKPGASVKVSCKASGYTFTNYYMYWVRQAPGQGLEWMGGINPSNGGTNFNE
KFKNRVTLTTDSSTTTAYMELKSLQFDDTAVYYCARRDYRFDMGFDYWGQGTTVTVSSGGGGS
GNWVNVISDLKKIEDLIQSMHIDATLYTESDVHPSCKVTAMKCFLLELQVISLESGDASIHDTVEN
LIILANNSLSSNGNVTESGCKECEELEEKNIKEFLQSFVHIVQMFINTSGGGGSASTKGPSVFPLAP
CSRSTSESTAALGCLVKDYFPEPVTVSWNSGALTSGVHTFPAVLQSSGLYSLSSVVTVPSSSLGTKT
YTCNVDHKPSNTKVDKRVESKYGPPCPPCPAPEFLGGPSVFLFPPKPKDTLMISRTPEVTCVVVDV
SQEDPEVQFNWYVDGVEVHNAKTKPREEQFNSTYRVVSVLTVLHQDWLNGKEYKCKVSNKGL
PSSIEKTISKAKGQPREPQVYTLPPSQEEMTKNQVSLTCLVKGFYPSDIAVEWESNGQPENNYKTT
PPVLDSDGSFFLYSRLTVDKSRWQEGNVFSCSVMHEALHNHYTQKSLSLSLGK
SEQ ID NO:94 aPD1-16GS-IL15RaSUSHI-10GS-CL 
EIVLTQSPATLSLSPGERATLSCRASQSVSSYLAWYQQKPGQAPRLLIYDASKRATGIPARFSGSGS
GTDFTLTISSLEPEDFAVYYCDQRNNWPLTFGGGTKVEIKGGGGSGGGGSGGGGSGITCPPPMSV
EHADIWVKSYSLYSRERYICNSGFKRKAGTSSLTECVLNKATNVAHWTTPSLKCIRGGGGSGGGG
SRTVAAPSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC
SEQ ID NO:95 aPD1-16GS-IL15WT-10GS-CH1-Fc(null) 
QVQLVQSGAEVKKPGASVKVSCKASGYTFTAQYMHWVRQAPGQGLEWMGIINPSGGETGYAQ
KFQGRVTMTRDTSTSTVYMELSSLRSEDTAVYYCAKEGVADGYGLVDVWGQGTMVTVSSGGG
GSGGGGSGGGGSGNWVNVISDLKKIEDLIQSMHIDATLYTESDVHPSCKVTAMKCFLLELQVISL
 
ESGDASIHDTVENLIILANNSLSSNGNVTESGCKECEELEEKNIKEFLQSFVHIVQMFINTSGGGGS
GGGGSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPPVAGPSVFLFP
PKPKDTLMISRTPEVTCVVVDVSHEDPEVKFNWYVDGVEVHNAKTKPREEQYNSTYRVVSVLT
VLHQDWLNGKEYKCKVSNKGLPSSIEKTISKAKGQPREPQVYTLPPSRDELTKNQVSLTCLVKGF
YPSDIAVEWESNGQPENNYKTTPPVLDSDGSFFLYSKLTVDKSRWQQGNVFSCSVMHEALHNHY
TQKSLSLSPGK
SEQ ID NO:96 aPD1-11GS-IL15RaSUSHI-10GS-CL 
EIVLTQSPATLSLSPGERATLSCRASQSVSSYLAWYQQKPGQAPRLLIYDASKRATGIPARFSGSGS
GTDFTLTISSLEPEDFAVYYCDQRNNWPLTFGGGTKVEIKGGGGSGGGGSGITCPPPMSVEHADI
WVKSYSLYSRERYICNSGFKRKAGTSSLTECVLNKATNVAHWTTPSLKCIRGGGGSGGGGSRTVA
APSVFIFPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC
SEQ ID NO:97 aPD1-11GS-IL15WT-10GS-CH1-Fc(null) 
QVQLVQSGAEVKKPGASVKVSCKASGYTFTAQYMHWVRQAPGQGLEWMGIINPSGGETGYAQ
KFQGRVTMTRDTSTSTVYMELSSLRSEDTAVYYCAKEGVADGYGLVDVWGQGTMVTVSSGGG
GSGGGGSGNWVNVISDLKKIEDLIQSMHIDATLYTESDVHPSCKVTAMKCFLLELQVISLESGDAS
IHDTVENLIILANNSLSSNGNVTESGCKECEELEEKNIKEFLQSFVHIVQMFINTSGGGGSGGGGSA
STKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPPVAGPSVFLFPPKPKDTL
MISRTPEVTCVVVDVSHEDPEVKFNWYVDGVEVHNAKTKPREEQYNSTYRVVSVLTVLHQDWL
NGKEYKCKVSNKGLPSSIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSLS
PGK
SEQ ID NO:98 aPD1-6GS-IL15RaSUSHI-5GS-CL 
EIVLTQSPATLSLSPGERATLSCRASQSVSSYLAWYQQKPGQAPRLLIYDASKRATGIPARFSGSGS
GTDFTLTISSLEPEDFAVYYCDQRNNWPLTFGGGTKVEIKGGGGSGITCPPPMSVEHADIWVKSYS
LYSRERYICNSGFKRKAGTSSLTECVLNKATNVAHWTTPSLKCIRGGGGSRTVAAPSVFIFPPSDEQ
LKSGTASVVCLLNNFYPREAKVQWKVDNALQSGNSQESVTEQDSKDSTYSLSSTLTLSKADYEK
HKVYACEVTHQGLSSPVTKSFNRGEC
SEQ ID NO:99 aPD1-6GS-IL15WT-5GS-CH1-Fc(null) 
QVQLVQSGAEVKKPGASVKVSCKASGYTFTAQYMHWVRQAPGQGLEWMGIINPSGGETGYAQ
KFQGRVTMTRDTSTSTVYMELSSLRSEDTAVYYCAKEGVADGYGLVDVWGQGTMVTVSSGGG
GSGNWVNVISDLKKIEDLIQSMHIDATLYTESDVHPSCKVTAMKCFLLELQVISLESGDASIHDTV
ENLIILANNSLSSNGNVTESGCKECEELEEKNIKEFLQSFVHIVQMFINTSGGGGSASTKGPSVFPL
APSSKSTSGGTAALGCLVKDYFPEPVTVSWNSGALTSGVHTFPAVLQSSGLYSLSSVVTVPSSSLG
TQTYICNVNHKPSNTKVDKKVEPKSCDKTHTCPPCPAPPVAGPSVFLFPPKPKDTLMISRTPEVTC
VVVDVSHEDPEVKFNWYVDGVEVHNAKTKPREEQYNSTYRVVSVLTVLHQDWLNGKEYKCK
VSNKGLPSSIEKTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSLSLSPGK
SEQ ID NO:100 aPD1-2-HC-IgG4 
QVQLVQSGVEVKKPGASVKVSCKASGYTFTNYYMYWVRQAPGQGLEWMGGINPSNGGTNFNE
KFKNRVTLTTDSSTTTAYMELKSLQFDDTAVYYCARRDYRFDMGFDYWGQGTTVTVSSASTKGP
SVFPLAPCSRSTSESTAALGCLVKDYFPEPVTVSWNSGALTSGVHTFPAVLQSSGLYSLSSVVTVPS
SSLGTKTYTCNVDHKPSNTKVDKRVESKYGPPCPPCPAPEFLGGPSVFLFPPKPKDTLMISRTPEV
TCVVVDVSQEDPEVQFNWYVDGVEVHNAKTKPREEQFNSTYRVVSVLTVLHQDWLNGKEYKC
KVSNKGLPSSIEKTISKAKGQPREPQVYTLPPSQEEMTKNQVSLTCLVKGFYPSDIAVEWESNGQP
ENNYKTTPPVLDSDGSFFLYSRLTVDKSRWQEGNVFSCSVMHEALHNHYTQKSLSLSLGK
SEQ ID NO:101 aPD1-2-LC 
EIVLTQSPATLSLSPGERATLSCRASKGVSTSGYSYLHWYQQKPGQAPRLLIYLASYLESGVPARFS
GSGSGTDFTLTISSLEPEDFAVYYCQHSRDLPLTFGGGTKVEIKRTVAAPSVFIFPPSDEQLKSGTAS
VVCLLNNFYPREAKVQWKVDNALQSGNSQESVTEQDSKDSTYSLSSTLTLSKADYEKHKVYAC
EVTHQGLSSPVTKSFNRGEC
SEQ ID NO:102 aPD1-2 VH-hIL15-HC-IgG4 
QVQLVQSGVEVKKPGASVKVSCKASGYTFTNYYMYWVRQAPGQGLEWMGGINPSNGGTNFNE
KFKNRVTLTTDSSTTTAYMELKSLQFDDTAVYYCARRDYRFDMGFDYWGQGTTVTVSSGGGGS
GGGGSGGGGSGNWVNVISDLKKIEDLIQSMHIDATLYTESDVHPSCKVTAMKCFLLELQVISLES
GDASIHDTVENLIILANDSLSSNGNVTESGCKECEELEEKNIKEFLQSFVHIVQMFINTSGGGGSGG
GGSASTKGPSVFPLAPCSRSTSESTAALGCLVKDYFPEPVTVSWNSGALTSGVHTFPAVLQSSGLY
SLSSVVTVPSSSLGTKTYTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQFNSTYRVVSVLTVLHQD
WLNGKEYKCKVSNKGLPSSIEKTISKAKGQPREPQVYTLPPSQEEMTKNQVSLTCLVKGFYPSDI
AVEWESNGQPENNYKTTPPVLDSDGSFFLYSRLTVDKSRWQEGNVFSCSVMHEALHNHYTQKSL
SLSLGK
SEQ ID NO:103 aPD1-2 VL-hIL15RaSushi-CL 
EIVLTQSPATLSLSPGERATLSCRASKGVSTSGYSYLHWYQQKPGQAPRLLIYLASYLESGVPARFS
GSGSGTDFTLTISSLEPEDFAVYYCQHSRDLPLTFGGGTKVEIKGGGGSGGGGSGGGGSGITCPPP
MSVEHADIWVKSYSLYSRERYICNSGFKRKAGTSSLTECVLNKATNVAHWTTPSLKCIRGGGGSG
GGGSKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO:104 aPD1-HC 
QVQLVQSGAEVKKPGASVKVSCKASGYTFTAQYMHWVRQAPGQGLEWMGIINPSGGETGYAQ
KFQGRVTMTRDTSTSTVYMELSSLRSEDTAVYYCAKEGVADGYGLVDVWGQGTMVTVSSASTK
GPSVFPLAPSSKSTSGGTAALGCLVKDYFPEPVTVSWNSGALTSGVHTFPAVLQSSGLYSLSSVVT
VPSSSLGTQTYICNVNHKPSNTKVDKKVEPKSCDKTHTCPPCPAPPVAGPSVFLFPPKPKDTLMIS
RTPEVTCVVVDVSHEDPEVKFNWYVDGVEVHNAKTKPREEQYNSTYRVVSVLTVLHQDWLNG
KEYKCKVSNKGLPSSIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHYTQKSLSLSPG
K
SEQ ID NO:105 aPD1-LC 
EIVLTQSPATLSLSPGERATLSCRASQSVSSYLAWYQQKPGQAPRLLIYDASKRATGIPARFSGSGS
GTDFTLTISSLEPEDFAVYYCDQRNNWPLTFGGGTKVEIKRTVAAPSVFIFPPSDEQLKSGTASVVC
 
LLNNFYPREAKVQWKVDNALQSGNSQESVTEQDSKDSTYSLSSTLTLSKADYEKHKVYACEVTH
QGLSSPVTKSFNRGEC
SEQ ID NO:106 aB7H3-2 VH 5GS-IL15WT-5GS-HC(hIgG1, wt) 
EVQLVQSGAEVKKPGASVKVSCKASGYTFTNYDINWVRQAPGQGLEWIGWIFPGDGSTQYNEK
FKGRATLTTDTSTSTAYMELRSLRSDDTAVYFCARQTTATWFAYWGQGTLVTVSSGGGGSNWVN
VISDLKKIEDLIQSMHIDATLYTESDVHPSCKVTAMKCFLLELQVISLESGDASIHDTVENLIILANN
SLSSNGNVTESGCKECEELEEKNIKEFLQSFVHIVQMFINTSGGGGSASTKGPSVFPLAPSSKSTSG
GTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYKCKVSNKALPAPI
EKTISKAKGQPREPQVYTLPPSREEMTKNQVSLTCLVKGFYPSDIAVEWESNGQPENNYKTTPPV
LDSDGSFFLYSKLTVDKSRWQQGNVFSCSVMHEALHNHYTQKSLSLSPGK
SEQ ID NO:107 aB7H3-2 VL-5GS-hIL15RaSUSHI-5GS-LC(hKappa) 
DIVMTQSPLSLPVTPGEPASISCRASQSISDYLHWYLQKPGESPRLLIKYASQSISGIPDRFSGSGSG
SDFTLKISRVEAEDVGVYYCQNGHSFPLTFGAGTKLEIKGGGGSITCPPPMSVEHADIWVKSYSLY
SRERYICNSGFKRKAGTSSLTECVLNKATNVAHWTTPSLKCIRGGGGSKRTVAAPSVFIFPPSDEQL
KSGTASVVCLLNNFYPREAKVQWKVDNALQSGNSQESVTEQDSKDSTYSLSSTLTLSKADYEKH
KVYACEVTHQGLSSPVTKSFNRGEC
SEQ ID NO:108 aB7H3-2 VH-5GS-IL15WT-5GS-HC(hIgG1, Null) 
EVQLVQSGAEVKKPGASVKVSCKASGYTFTNYDINWVRQAPGQGLEWIGWIFPGDGSTQYNEK
FKGRATLTTDTSTSTAYMELRSLRSDDTAVYFCARQTTATWFAYWGQGTLVTVSSGGGGSNWVN
VISDLKKIEDLIQSMHIDATLYTESDVHPSCKVTAMKCFLLELQVISLESGDASIHDTVENLIILANN
SLSSNGNVTESGCKECEELEEKNIKEFLQSFVHIVQMFINTSGGGGSASTKGPSVFPLAPSSKSTSG
GTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPPVAGPSVFLFPPKPKDTLMISRTPEVTCVVVDVSHED
PEVKFNWYVDGVEVHNAKTKPREEQYNSTYRVVSVLTVLHQDWLNGKEYKCKVSNKGLPSSIE
KTISKAKGQPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
SEQ ID NO:109 aPD1-3-kLC 
EIVLTQSPATLSLSPGERATLSCRASQSVSSYLAWYQQKPGQAPRLLIYDASNRATGIPARFSGSGS
GTDFTLTISSLEPEDFAVYYCQQSSNWPRTFGQGTKVEIKRTVAAPSVFIFPPSDEQLKSGTASVVC
LLNNFYPREAKVQWKVDNALQSGNSQESVTEQDSKDSTYSLSSTLTLSKADYEKHKVYACEVTH
QGLSSPVTKSFNRGEC
SEQ ID NO:110 aPD1-3-HC-IgG4 
QVQLVESGGGVVQPGRSLRLDCKASGITFSNSGMHWVRQAPGKGLEWVAVIWYDGSKRYYADS
VKGRFTISRDNSKNTLFLQMNSLRAEDTAVYYCATNDDYWGQGTLVTVSSASTKGPSVFPLAPCS
RSTSESTAALGCLVKDYFPEPVTVSWNSGALTSGVHTFPAVLQSSGLYSLSSVVTVPSSSLGTKTY
TCNVDHKPSNTKVDKRVESKYGPPCPPCPAPEFLGGPSVFLFPPKPKDTLMISRTPEVTCVVVDVS
QEDPEVQFNWYVDGVEVHNAKTKPREEQFNSTYRVVSVLTVLHQDWLNGKEYKCKVSNKGLP
SSIEKTISKAKGQPREPQVYTLPPSQEEMTKNQVSLTCLVKGFYPSDIAVEWESNGQPENNYKTTP
PVLDSDGSFFLYSRLTVDKSRWQEGNVFSCSVMHEALHNHYTQKSLSLSLGK
SEQ ID NO:111 aPD1-3 HC-IgG1(null) 
QVQLVESGGGVVQPGRSLRLDCKASGITFSNSGMHWVRQAPGKGLEWVAVIWYDGSKRYYADS
VKGRFTISRDNSKNTLFLQMNSLRAEDTAVYYCATNDDYWGQGTLVTVSSASTKGPSVFPLAPSS
KSTSGGTAALGCLVKDYFPEPVTVSWNSGALTSGVHTFPAVLQSSGLYSLSSVVTVPSSSLGTQTY
ICNVNHKPSNTKVDKKVEPKSCDKTHTCPPCPAPPVAGPSVFLFPPKPKDTLMISRTPEVTCVVVD
VSHEDPEVKFNWYVDGVEVHNAKTKPREEQYNSTYRVVSVLTVLHQDWLNGKEYKCKVSNKG
LPSSIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGK
SEQ ID NO:112 aPD1-3 VL-16GS-IL15RaSUSHI-10GS-LC 
EIVLTQSPATLSLSPGERATLSCRASQSVSSYLAWYQQKPGQAPRLLIYDASNRATGIPARFSGSGS
GTDFTLTISSLEPEDFAVYYCQQSSNWPRTFGQGTKVEIKGGGGSGGGGSGGGGSGITCPPPMSVE
HADIWVKSYSLYSRERYICNSGFKRKAGTSSLTECVLNKATNVAHWTTPSLKCIRGGGGSGGGGS
RTVAAPSVFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC
SEQ ID NO:113 aPD1-3 VH-16GS-IL15WT-10GS-HC-IgG4 
QVQLVESGGGVVQPGRSLRLDCKASGITFSNSGMHWVRQAPGKGLEWVAVIWYDGSKRYYADS
VKGRFTISRDNSKNTLFLQMNSLRAEDTAVYYCATNDDYWGQGTLVTVSSGGGGSGGGGSGGG
GSGNWVNVISDLKKIEDLIQSMHIDATLYTESDVHPSCKVTAMKCFLLELQVISLESGDASIHDTV
ENLIILANNSLSSNGNVTESGCKECEELEEKNIKEFLQSFVHIVQMFINTSGGGGSGGGGSASTKG
PSVFPLAPCSRSTSESTAALGCLVKDYFPEPVTVSWNSGALTSGVHTFPAVLQSSGLYSLSSVVTVP
SSSLGTKTYTCNVDHKPSNTKVDKRVESKYGPPCPPCPAPEFLGGPSVFLFPPKPKDTLMISRTPE
VTCVVVDVSQEDPEVQFNWYVDGVEVHNAKTKPREEQFNSTYRVVSVLTVLHQDWLNGKEYK
CKVSNKGLPSSIEKTISKAKGQPREPQVYTLPPSQEEMTKNQVSLTCLVKGFYPSDIAVEWESNGQ
PENNYKTTPPVLDSDGSFFLYSRLTVDKSRWQEGNVFSCSVMHEALHNHYTQKSLSLSLGK
SEQ ID NO:114 aPD1-3 VH-16GS-IL15WT-10GS-HC-IgG1(null) 
QVQLVESGGGVVQPGRSLRLDCKASGITFSNSGMHWVRQAPGKGLEWVAVIWYDGSKRYYADS
VKGRFTISRDNSKNTLFLQMNSLRAEDTAVYYCATNDDYWGQGTLVTVSSGGGGSGGGGSGGG
GSGNWVNVISDLKKIEDLIQSMHIDATLYTESDVHPSCKVTAMKCFLLELQVISLESGDASIHDTV
ENLIILANNSLSSNGNVTESGCKECEELEEKNIKEFLQSFVHIVQMFINTSGGGGSGGGGSASTKG
PSVFPLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPPVAGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKGLPSSIEKTISKAKGQPREPQVYTLPPSRDELTKNQVSLTCLVKGFYPSDIAVEWESN
GQPENNYKTTPPVLDSDGSFFLYSKLTVDKSRWQQGNVFSCSVMHEALHNHYTQKSLSLSPGK
SEQ ID NO:115 aPD1-3 VL-16GS-IL15WT-10GS-LC 
EIVLTQSPATLSLSPGERATLSCRASQSVSSYLAWYQQKPGQAPRLLIYDASNRATGIPARFSGSGS
GTDFTLTISSLEPEDFAVYYCQQSSNWPRTFGQGTKVEIKGGGGSGGGGSGGGGSGNWVNVISDL
KKIEDLIQSMHIDATLYTESDVHPSCKVTAMKCFLLELQVISLESGDASIHDTVENLIILANNSLSSN
GNVTESGCKECEELEEKNIKEFLQSFVHIVQMFINTSGGGGSGGGGSRTVAAPSVFIFPPSDEQLKS
GTASVVCLLNNFYPREAKVQWKVDNALQSGNSQESVTEQDSKDSTYSLSSTLTLSKADYEKHKV
YACEVTHQGLSSPVTKSFNRGEC
SEQ ID NO:116 aPD1-3 VH-16GS-IL15RaSushi-10GS-HC-IgG4 
QVQLVESGGGVVQPGRSLRLDCKASGITFSNSGMHWVRQAPGKGLEWVAVIWYDGSKRYYADS
VKGRFTISRDNSKNTLFLQMNSLRAEDTAVYYCATNDDYWGQGTLVTVSSGGGGSGGGGSGGG
GSGITCPPPMSVEHADIWVKSYSLYSRERYICNSGFKRKAGTSSLTECVLNKATNVAHWTTPSLKC
IRGGGGSGGGGSASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPPCPAPEFLGGPSV
FLFPPKPKDTLMISRTPEVTCVVVDVSQEDPEVQFNWYVDGVEVHNAKTKPREEQFNSTYRVVS
VLTVLHQDWLNGKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNVFSCSVMHEALH
NHYTQKSLSLSLGK
SEQ ID NO:117 aPD1-3 VL-16GS-IL15RaSushi-10GS-HC-IgG1(null)

QVQLVESGGGVVQPGRSLRLDCKASGITFSNSGMHWVRQAPGKGLEWVAVIWYDGSKRYYADS
VKGRFTISRDNSKNTLFLQMNSLRAEDTAVYYCATNDDYWGQGTLVTVSSGGGGSGGGGSGGG
GSGITCPPPMSVEHADIWVKSYSLYSRERYICNSGFKRKAGTSSLTECVLNKATNVAHWTTPSLKC
IRGGGGSGGGGSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPAPPVAGP
SVFLFPPKPKDTLMISRTPEVTCVVVDVSHEDPEVKFNWYVDGVEVHNAKTKPREEQYNSTYRV
VSVLTVLHQDWLNGKEYKCKVSNKGLPSSIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEA
LHNHYTQKSLSLSPGK

In a second typical embodiment of the present application, a DNA molecule is provided, which encodes the aforementioned fusion antibody.

In a third typical embodiment of the present application, a recombinant plasmid is provided, which is ligated with the aforementioned DNA molecule.

In a fourth typical embodiment of the present application, a host cell is provided, which is transformed with the aforementioned recombinant plasmid. In a preferred embodiment, the host cell is a prokaryotic cell or a eukaryotic cell. Specifically, the prokaryotic cell may be Escherichia coli; the eukaryotic cell may be a yeast cell, a mammalian cell, an insect cell, or the like.

In a fifth typical embodiment of the present application, a use of the aforementioned fusion antibody in the manufacture of a medicament for the prevention and/or treatment of cancer is provided. The fusion antibody can effectively target the tumor microenvironment, specifically kill tumors, avoid systemic overactivation of NK cells inducing immunotoxicity, and has a long half-life, thereby achieving favorable effects in future tumor prevention and treatment.

In a preferred embodiment, the cancer includes any of the following: lung cancer, melanoma, colon cancer, rectal cancer, liver cancer, lymphoma, breast cancer, prostate cancer, or hematological tumor.

In a sixth typical embodiment of the present application, a medicament is provided, comprising the aforementioned fusion antibody.

Given the strong cellular targeting and long half-life of the fusion antibody of the present application, combined use with an immune checkpoint inhibitor can achieve better synergistic therapeutic effects. In a preferred embodiment, the medicament further comprises an immune checkpoint inhibitor used in combination with the fusion antibody, wherein the immune checkpoint inhibitor includes any one or more of the following: aPDL1 (anti-PDL1 antibody), aCTLA4 (anti-CTLA4 antibody), a41BB (anti-41BB antibody), or aLAG3 (anti-LAG3 antibody).

In a seventh typical embodiment of the present application, a method for treating cancer is provided, comprising: administering to a subject an effective amount of the aforementioned fusion antibody or the aforementioned medicament. The aforementioned fusion antibody or medicament can effectively target the tumor microenvironment, specifically kill tumors, avoid immunotoxicity induced by systemic overactivation of T cells or NK cells, and has a long half-life, thereby achieving favorable effects in future tumor prevention and treatment. Combination with other drugs enhances the killing function of tumor-killing cells in the tumor microenvironment, promotes the activation and proliferation of tumor-killing cells, enables the combined medicament to exert better functional activity in the tumor microenvironment and achieves better synergistic therapeutic effects.

In a preferred embodiment, the cancer includes any of the following: lung cancer, melanoma, colon cancer, rectal cancer, liver cancer, lymphoma, breast cancer, prostate cancer, or hematological tumor.

The present application is further described in detail below with reference to specific examples, which should not be construed as limiting the scope of protection claimed by the present application. Unless otherwise specified, reagents or consumables used in the following examples were purchased from commercial sources.

### Examples

### Example 1. Construction of Eukaryotic Expression Vectors for Cytokine Fusion Antibodies

Gene fragments of VL and VH of antibodies against EGFR, PDL1, PSMA, B7H3 and PD1, gene fragment of IL-15, gene fragment of sushi domain of IL-15Rα, and gene fragments of constant regions of antibodies against PDL1, PSMA and B7H3 were synthesized respectively.

The above gene fragments were amplified by PCR. The amplified VH gene fragment was linked with IL-15 gene fragment or IL-15Rα sushi domain gene fragment and antibody constant region gene fragment via a linker by overlap PCR to obtain VH-IL-15-CH1-Fc fragment or VH-IL-15Rα-CH1-Fc fragment, serving as the first chain (a different description of the same structure as the aforementioned heavy chain). The amplified VL gene fragment was linked with IL-15Rα gene fragment or IL-15 gene fragment and antibody constant region gene fragment via a linker to obtain VL-IL-15Rα-CL fragment, VL-IL-15-CL-Fc fragment, VL-IL-15-CL fragment or VL-IL-15Rα-CL-Fc fragment, serving as the second chain (a different description of the same structure as the aforementioned light chain). The above fragments were further ligated into the pFuse vector for eukaryotic expression (InvivoGen, CA) by homologous recombination, and transformed into Escherichia coli competent DH5α cells. Antibiotic selection was performed on LB plates, positive clones were selected, plasmids were extracted using an endotoxin-free plasmid extraction kit, and the sequences of the extracted plasmids were verified by sequencing. The nucleotide and amino acid sequences of each construct are listed in the Sequence Listing.

**Table 1: Fusion Protein Sequence Numbers**

| Fusion antibody | Amino acid sequence | |
|---|---|---|
| FuAb1 | First chain | SEQ ID NO:1 |
| | Second chain | SEQ ID NO:2 |
| FuAb2 | First chain | SEQ ID NO:3 |
| | Second chain | SEQ ID NO:4 |
| FuAb3 | First chain | SEQ ID NO:1 |
| | Second chain | SEQ ID NO:5 |
| FuAb4 | First chain | SEQ ID NO:1 |
| | Second chain | SEQ ID NO:6 |
| FuAb5 | First chain | SEQ ID NO:7 |
| | Second chain | SEQ ID NO:8 |
| FuAb6 | First chain | SEQ ID NO:9 |
| | Second chain | SEQ ID NO:10 |
| FuAb7 | First chain | SEQ ID NO:11 |
| | Second chain | SEQ ID NO:5 |
| FuAb8 | First chain | SEQ ID NO:45 |
| | Second chain | SEQ ID NO:46 |
| FuAb9 | First chain | SEQ ID NO:47 |
| | Second chain | SEQ ID NO:48 |
| FuAb10 | First chain | SEQ ID NO:49 |
| | Second chain | SEQ ID NO:50 |
| FuAb11 | First chain | SEQ ID NO:51 |
| | Second chain | SEQ ID NO:52 |
| FuAb12 | First chain | SEQ ID NO:55 |
| | Second chain | SEQ ID NO:56 |
| FuAb13 | First chain | SEQ ID NO:53 |
| | Second chain | SEQ ID NO:54 |
| FuAb14 | First chain | SEQ ID NO:57 |
| | Second chain | SEQ ID NO:58 |
| FuAb15 | First chain | SEQ ID NO:59 |
| | Second chain | SEQ ID NO:60 |
| FuAb16 | First chain | SEQ ID NO:57 |
| | Second chain | SEQ ID NO:60 |
| FuAb17 | First chain | SEQ ID NO:67 |
| | Second chain | SEQ ID NO:68 |
| FuAb18 | First chain | SEQ ID NO:69 |
| | Second chain | SEQ ID NO:70 |
| FuAbl9 | First chain | SEQ ID NO:71 |
| | Second chain | SEQ ID NO:72 |
| FuAb20 | First chain | SEQ ID NO:73 |
| | Second chain | SEQ ID NO:74 |
| FuAb21 | First chain | SEQ ID NO:75 |
| | Second chain | SEQ ID NO:76 |
| FuAb22 | First chain | SEQ ID NO:77 |
| | Second chain | SEQ ID NO:78 |
| FuAb23 | First chain | SEQ ID NO:82 |
| | Second chain | SEQ ID NO:8 |
| FuAb24 | First chain | SEQ ID NO:83 |
| | Second chain | SEQ ID NO:84 |
| FuAb25 | First chain | SEQ ID NO:85 |
| | Second chain | SEQ ID NO:84 |
| FuAb26 | First chain | SEQ ID NO:83 |
| | Second chain | SEQ ID NO:86 |
| FuAb27 | First chain | SEQ ID NO:85 |
| | Second chain | SEQ ID NO:86 |
| FuAb29 | First chain | SEQ ID NO:88 |
| | Second chain | SEQ ID NO:89 |
| FuAb30 | First chain | SEQ ID NO:90 |
| | Second chain | SEQ ID NO:91 |
| FuAb31 | First chain | SEQ ID NO:92 |
| | Second chain | SEQ ID NO:93 |
| FuAb32 | First chain | SEQ ID NO:94 |
| | Second chain | SEQ ID NO:95 |
| FuAb33 | First chain | SEQ ID NO:96 |
| | Second chain | SEQ ID NO:97 |
| FuAb34 | First chain | SEQ ID NO:98 |
| | Second chain | SEQ ID NO:99 |
| FuAb36 | First chain | SEQ ID NO:102 |
| | Second chain | SEQ ID NO:103 |
| FuAb38 | First chain | SEQ ID NO:106 |
| | Second chain | SEQ ID NO:107 |
| FuAb39 | First chain | SEQ ID NO:108 |
| | Second chain | SEQ ID NO:107 |
| FuAb40 | First chain | SEQ ID NO:85 |
| | Second chain | SEQ ID NO:84 |
| FuAb44 | First chain | SEQ ID NO:112 |
| | Second chain | SEQ ID NO:113 |
| FuAb45 | First chain | SEQ ID NO:112 |
| | Second chain | SEQ ID NO:114 |
| FuAb46 | First chain | SEQ ID NO:115 |
| | Second chain | SEQ ID NO:116 |
| FuAb47 | First chain | SEQ ID NO:115 |
| | Second chain | SEQ ID NO:117 |

### Example 2. Expression and Purification of Cytokine Fusion Antibodies

The eukaryotic expression vector plasmids carrying the heavy and light chains of the cytokine-fusion antibodies constructed in Example 1 were co-transfected into FreeStyle HEK293 cells and cultured under conditions of 125 rpm, 37°C, 5% CO₂ for 5-6 days. The cell culture supernatant was collected by centrifugation, filtered through a 0.22 µm filter, and the fusion antibodies were purified using Protein A Resin (Genscript) according to the manufacturer's instructions. Concentrations were determined by A280 and BCA (Pierce).

The fusion antibodies purified by Protein A resin were further separated, purified, and buffer-exchanged using a GE AKTA chromatography system and a Superdex 200 Increase 10/300 GL gel filtration column in PBS buffer (pH 7.4). The purified samples were stored in PBS buffer (pH 7.4). The composition and purity of the cytokine-fusion antibodies were assessed by SDS-PAGE under reducing and non-reducing conditions. The monomeric component of the cytokine-fusion antibodies in a saline solution environment was analyzed by size exclusion chromatography (SEC).

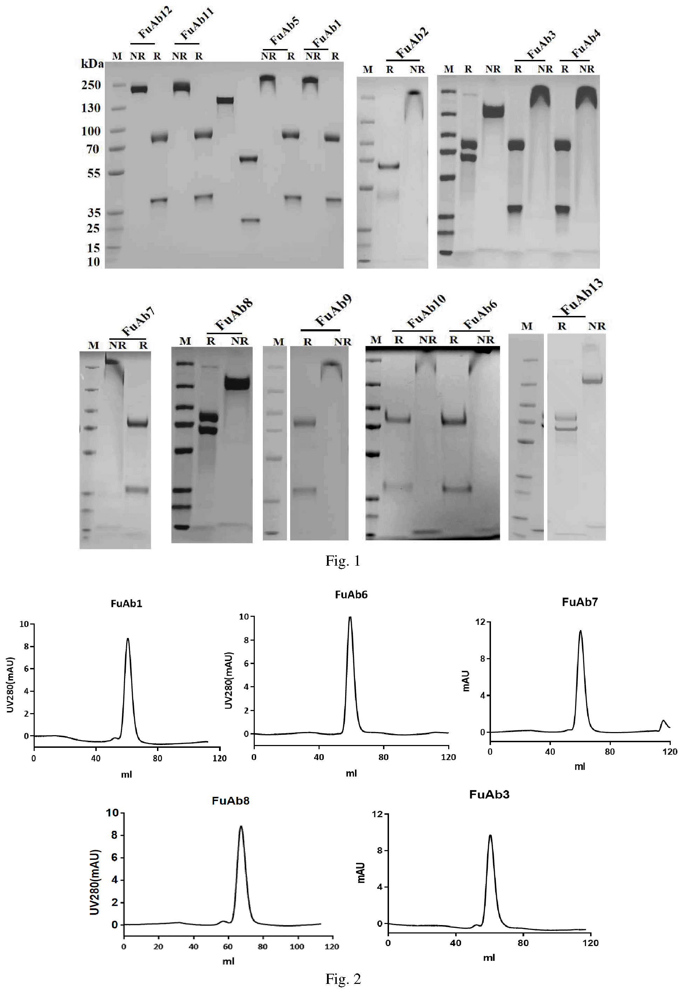

The results are shown in Figure 1, where R indicates the band after adding reducing agent (i.e., the first and second chains), and NR indicates the band without reducing agent (i.e., the fusion antibody). The size of the fusion antibody shown in the NR lane was as expected. As shown in Figure 2, in SEC analysis, the monomer peak of the fusion antibody accounted for >95% with no significant aggregates or fragments, demonstrating expression and purification advantages similar to antibodies.

### Example 3. Mass Spectrometric Analysis of Cytokine Fusion Antibodies

Samples purified in Example 2 (FuAb2, FuAb1, FuAb7, FuAb6, FuAb5, FuAb8, FuAb23, FuAb17, FuAb18 and FuAb19) were incubated with PNGase F (NEB) at 37°C overnight at a concentration of 1 mg/mL. The deglycosylated sample was reduced by adding 10 mM DTT, and the reduced sample was injected into a 300SB-C8, 2.1 × 50 mm column of HPLC-Q-TOF-MS (Agilent, USA) for MS analysis. The results are shown in Table 2. The theoretically predicted molecular weights of the two chains of different cytokine fusion antibodies are basically consistent with the molecular weights detected by mass spectrometry.

**Table 2 MS analysis**

| | | Theoretical Molecular Weight | Detected Molecular Weight |
|---|---|---|---|
| FuAb2 | First chain | 63076.76 | 63069.21 |
| | Second chain | 32822.66 | 32814.75 |
| FuAb1 | First chain | 63076.76 | 63071.74 |
| | Second chain | 32327.98 | 32321.35 |
| FuAb7 | First chain | 63571.44 | 63564.77 |
| | Second chain | 32822.66 | 32815.51 |
| FuAb6 | First chain | 63293.03 | 63268.80 |
| | Second chain | 32443.07 | 32435.90 |
| FuAb5 | First chain | 62882.55 | 62879.79 |
| | Second chain | 32548.12 | 32543.48 |
| FuAb8 | First chain | 62896.57 | 62888.8 |
| | Second chain | 57677.7 | 57666.94 |
| FuAb23 | First chain | 62881.56 | 62877.76 |
| | Second chain | 32548.12 | 32542.04 |
| FuAb17 | First chain | 62509.23 | 62504.69 |
| | Second chain | 32175.78 | 32169.06 |
| FuAb18 | First chain | 62193.94 | 62188.85 |
| | Second chain | 31860.50 | 31853.51 |
| FuAb19 | First chain | 61878.66 | 61874.51 |
| | Second chain | 31545.21 | 31538.76 |

### Example 4. Thermodynamic Stability Testing

The fusion antibody at a concentration of 1 mg/mL was mixed with freshly prepared thermal shift dye and shift buffer (Protein Thermal Shift^{™} Dye Kit, ThermoFisher Scientific, Cat. 4461146) at the ratio recommended by the manufacturer, and thermal scanning was performed from 25°C to 99°C at a heating rate of 0.05°C/s using a ViiA^{™} 7 Real-Time PCR System. The melting temperature (Tm) was calculated using the "Area under curve (AUC)" analysis model of GraphPad Prism 7 software.

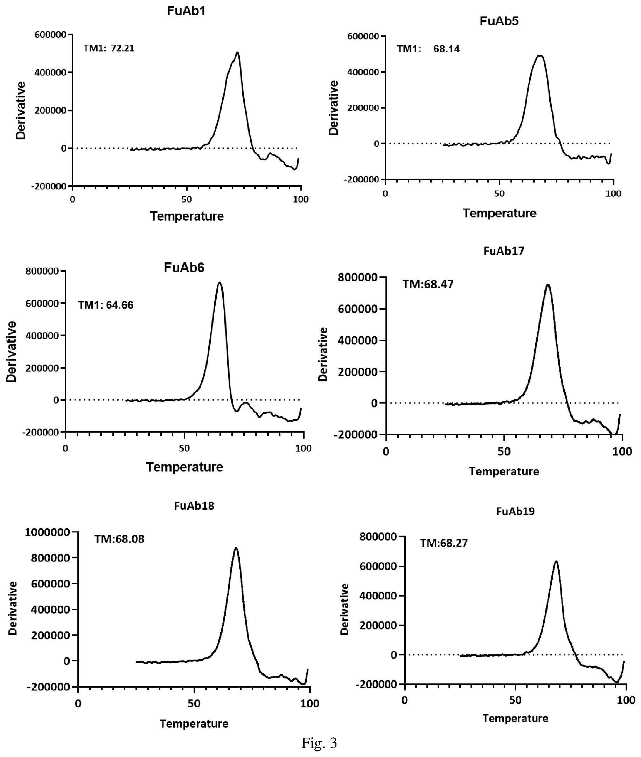

The results are shown in Figure 3. The melting temperatures of fusion antibody samples FuAb1, FuAb5, FuAb6, FuAb17, FuAb18 and FuAb19 fused with three different antibodies (PDL1, PSMA, B7H3) are in the range of 65-75°C, all higher than the first melting temperature Tm1 = 60.79°C of the parental antibody, indicating that the cytokine fusion antibodies have good thermodynamic stability.

### Example 5. Binding Activity Assays

### 5.1 ELISA Assay

Target antigen (100 ng/100 µL/well) was coated on a 96-well ELISA plate and incubated overnight at 4°C. After blocking with PBST containing 2% skim milk (0.5% Tween-20 in PBS) at room temperature for 1 hour, washing was performed with PBST. The cytokine fusion antibody sample or the cytokine fusion antibody digested by MMP-2 (wherein MMP-2 can recognize and cleave the cleavage site in the linker peptide of the fusion antibody as set forth in SEQ ID NO: 62 or 65: between S and L of IPVSLRSG (as set forth in SEQ ID NO: 66)) (the specific digestion conditions are as follows: the fusion protein containing the cleavage site was replaced into a buffer containing 50 mM Tris, 10 mM CaCl₂, 100 mM NaCl, pH 7.4, MMP-2 was mixed with each fusion protein at a ratio of 1:40, and digested at 37°C for 24 h) (samples after digestion are indicated by the suffix -C after the fusion antibody) was diluted to 50 nM with blocking solution, and serially diluted 5-fold from this starting concentration for a total of 8 gradients. 100 µL of diluted cytokine fusion antibody sample was added to each well and incubated at room temperature for 2 h. After washing 3 times with PBST, 100 µL of HRP-labeled goat anti-human Fc monoclonal antibody diluted 1:500 according to the instructions was added to each well, incubated at room temperature for 1 h, and washed 5 times with PBST. 100 µL of freshly prepared TMB chromogenic reagent (BioLegend, Cat. 421101) was added to the washed ELISA wells, incubated at room temperature in the dark for 5-30 minutes, and read at 650 nm in a microplate reader after color development.

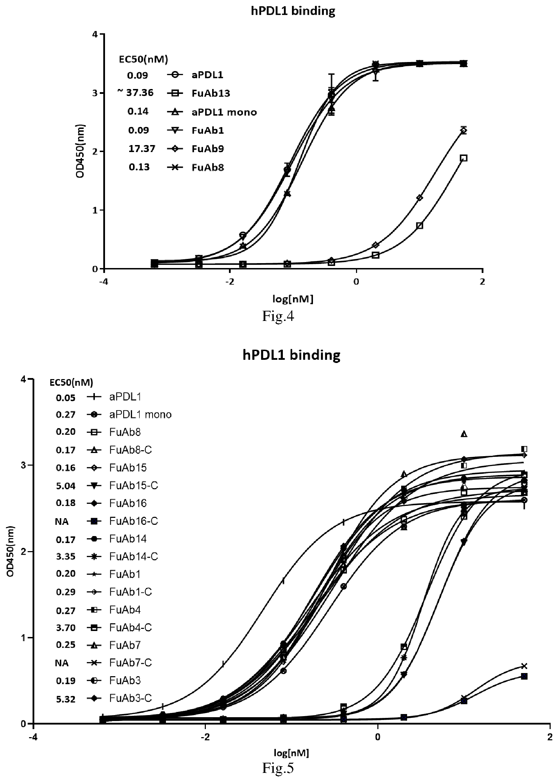

Monoclonal antibodies against target antigens aPDL1 (wherein the amino acid sequences of the heavy and light chains are as set forth in SEQ ID NO: 23 and SEQ ID NO: 24), aB7H3 (wherein the amino acid sequences of the heavy and light chains are as set forth in SEQ ID NO: 33 and SEQ ID NO: 34), aB7H3-2 (wherein the amino acid sequences of the heavy and light chains are as set forth in SEQ ID NO: 118 and SEQ ID NO: 119), aPD1 (wherein the amino acid sequences of the heavy and light chains are as set forth in SEQ ID NO: 138 and SEQ ID NO: 139), aPD1-2 (wherein the amino acid sequences of the heavy and light chains are as set forth in SEQ ID NO: 142 and SEQ ID NO: 143), aPD1-3 (wherein the amino acid sequences of the heavy and light chains are as set forth in SEQ ID NO: 140 and SEQ ID NO: 141) or aPDL1 mono (monovalent binding to antigen compared with bivalent aPDL1, wherein the amino acid sequences of the heavy and light chains are as set forth in SEQ ID NO: 23 and SEQ ID NO: 24 + Fc) were used as control samples. EC50 values were calculated using the "log(agonist) vs. response - Variable slope (four parameters)" analysis model of GraphPad Prism 7 software. The results are shown in Figures 4-6.

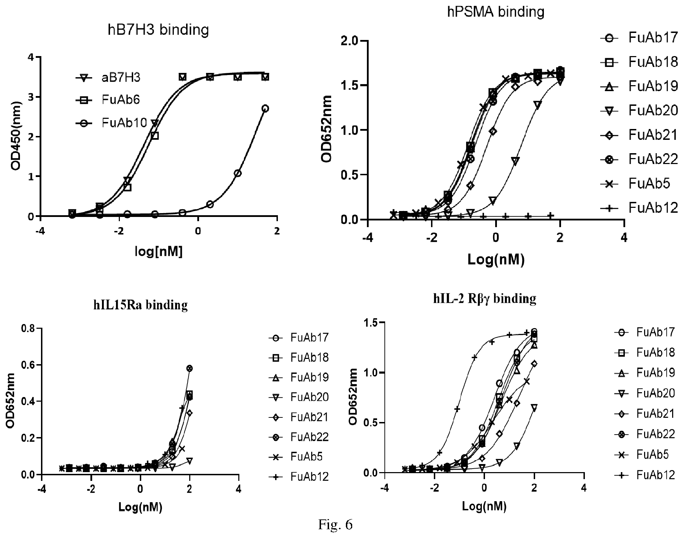

As shown in Figure 4, the ELISA binding activities of FuAb1 and FuAb8 to PDL1 antigen are similar to that of aPDL1, and stronger than those of fusion proteins FuAb9 and FuAb13 in which IL-15 and IL-15Rα are located at the N-termini of the heavy and light chain variable regions, respectively. In addition, as shown in Figure 6, the ELISA binding activity of FuAb6 to B7H3 antigen is consistent with that of aB7H3, and stronger than that of fusion protein FuAb10 in which IL-15 and IL-15Rα are located at the N-termini of the heavy and light chain variable regions, respectively; the ELISA binding activities of FuAb17-22 to PSMA antigen are consistent with that of FuAb5, and stronger than that of control FuAb12. It can be seen that the embedded fusion mode does not affect the binding activity of the N-terminal antigen-binding domain to the antigen. In addition, the binding abilities of FuAb5, FuAb17-22 to IL15Rα and IL2Rβγ are significantly decreased compared with that of FuAb12.

As shown in Figure 5, regarding the binding activity to PD-L1, the ELISA binding curves of fusion protein FuAb1 without cleavage site and digested sample FuAb8-C of FuAb8 show no obvious change compared with those of FuAb1 and FuAb8 samples. It can be seen that the ELISA antigen-binding activity of the fusion protein without cleavage site is not affected by protease; the ELISA binding curves of fusion proteins with a single cleavage site (e.g., FuAb3, FuAb4, FuAb14, FuAb15) shift to the right after digestion, indicating that the binding activity of the N-terminal antigen-binding domain to the antigen is reduced after single digestion; fusion proteins with double cleavage sites (e.g., FuAb7, FuAb16) show almost no binding in ELISA after digestion.

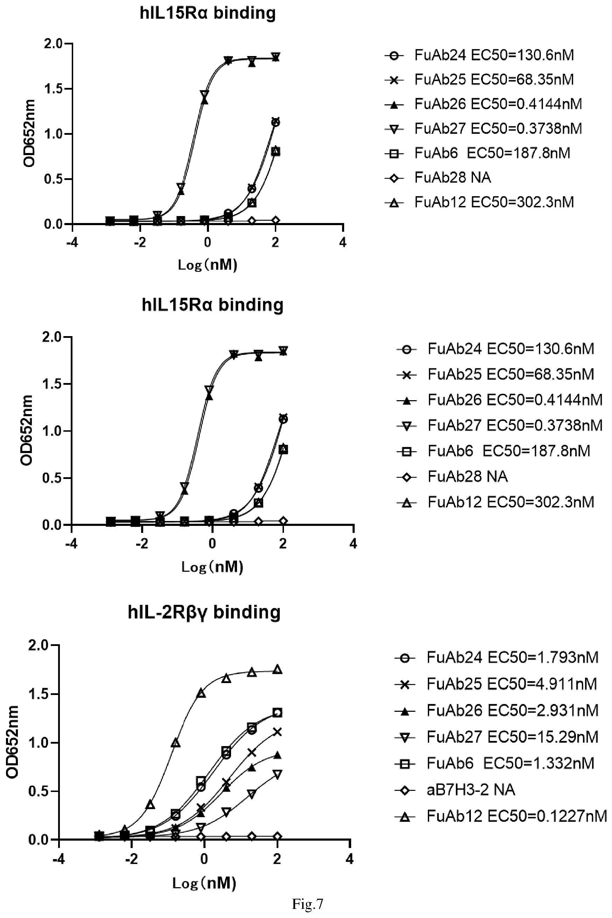

As shown in Figure 7, the ELISA binding activities of embedded fusion proteins FuAb24, 25 to B7H3 antigen are basically consistent with that of aB7H3 monoclonal antibody, and the binding ability is stronger than that of FuAb26, 27 in which IL-15 is chimerically fused unilaterally to the heavy and light chains, respectively; regarding the binding ability to IL-2Rβγ, the embedded fusion proteins FuAb24, 25, 26, 27 are all lower than that of sample FuAb12 (positive control, wherein IL-15 and IL-15Rα were respectively fused to the N-termini of the antibody heavy and light chains, respectively), with EC50 differences of 10-fold or more. Regarding IL15Rα binding ability, the embedded fusion proteins are all lower than that of FuAb26, 27 in which IL-15 is chimerically fused unilaterally to the heavy and light chains, respectively, with EC50 differences of 50-fold or more.

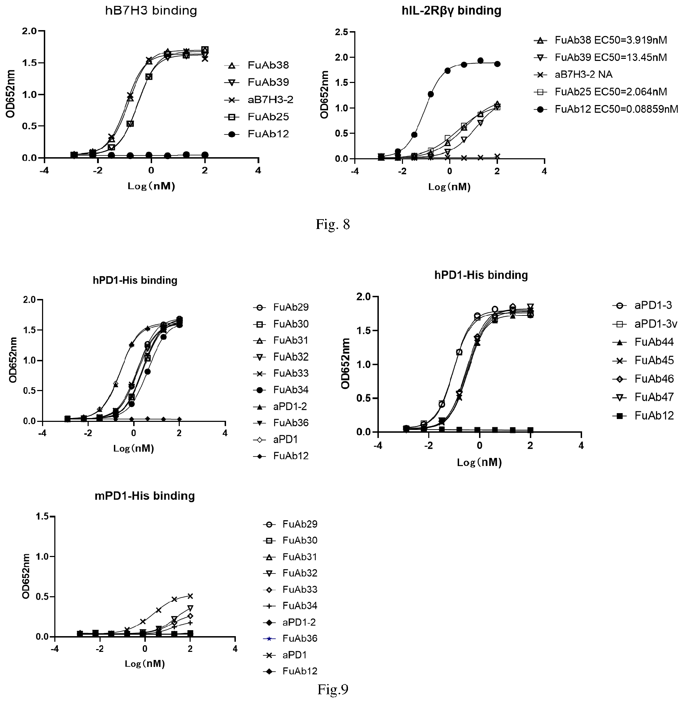

As shown in Figure 8, the ELISA binding activities of FuAb38, 39 to B7H3 antigen are basically consistent with that of aB7H3-2 monoclonal antibody, and the binding abilities to IL-2Rβγ are both lower than that of FuAb12, with EC50 differences of 40-fold or more.

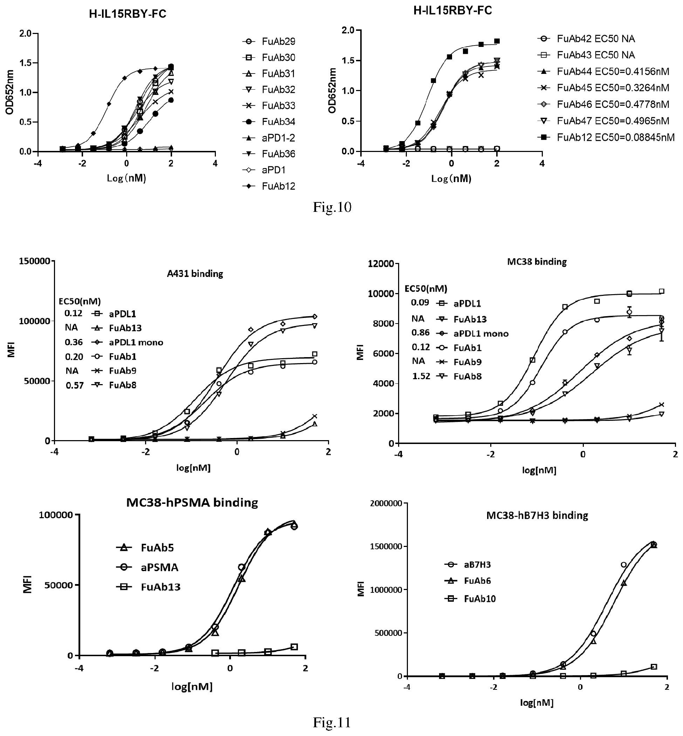

As shown in Figure 9, regarding PD1 binding ability, the ELISA binding activities of embedded fusion proteins FuAb29-34, FuAb44-47 to PD1 antigen are somewhat decreased compared with that of aPD1 monoclonal antibody (wherein M-PD1-His is murine PD1 antigen). As shown in Figure 10, their binding ability to IL-2Rβγ was lower than that of the positive control sample FuAb12, with EC₅₀ differences ranging from 4 to 25-fold.

### 5.2 Cell Surface Receptor Binding Activity Assay

A431 cells (naturally expressing human PDL1 antigen on the cell surface), MC38-hB7H3, MC38-hPSMA and MC38 cells (naturally expressing murine PDL1 antigen on the cell surface) were cultured, wherein MC38-hB7H3 and MC38-hPSMA are MC38 cells genetically engineered to express human B7H3 and PSMA antigens, respectively. All the above cells were adherently cultured in DMEM medium containing 10% FBS, and harvested after trypsin digestion. 2×10⁵ cells were aliquoted into each flow cytometry staining well, and blocked with pre-cooled 2% FBS-PBS blocking solution for 30 minutes. The cytokine fusion antibody sample (digested product or undigested) was diluted to 50 nM with blocking solution, and serially diluted 5-fold from this starting concentration for a total of 8 gradients. 0 nM concentration was set as the staining background. 100 µL of diluted cytokine fusion antibody sample was added to each well and incubated at 4°C for 30 minutes. After washing 3 times with PBS, 100 µL of APC-labeled anti-human Fc monoclonal antibody diluted according to the instructions was added to each well, incubated at 4°C for 30 minutes, and washed 3 times with PBS. 200 µL of blocking solution was added to the washed flow tube, and the resuspended cell suspension was used for flow cytometry analysis. EC50 values were calculated using the "log(agonist) vs. response -Variable slope (four parameters)" analysis model of GraphPad Prism 7 software.

Monoclonal antibodies against target antigens aPDL1, aB7H3, aPDL1 mono or aPSMA were used as control samples.

The results are shown in Figure 11. The binding activities of fusion proteins with IL15 and IL15Rα located between antibody variable and constant regions (e.g., FuAb1, FuAb5, FuAb6, FuAb8) to cell surface receptors are consistent with those of parental monoclonal antibodies against target antigens (aPDL1, aPSMA, aB7H3), and stronger than those of fusion proteins with IL15 and IL15Rα located at the N-termini of antibody variable regions (e.g., FuAb9, FuAb11, FuAb10 and FuAb13), indicating that the fusion of IL-15/IL-15Rα in the antibody of the present application (hereinafter referred to as "embedded fusion") does not affect the binding activity of the N-terminal VH/VL antigen-binding domain to cell surface receptors.

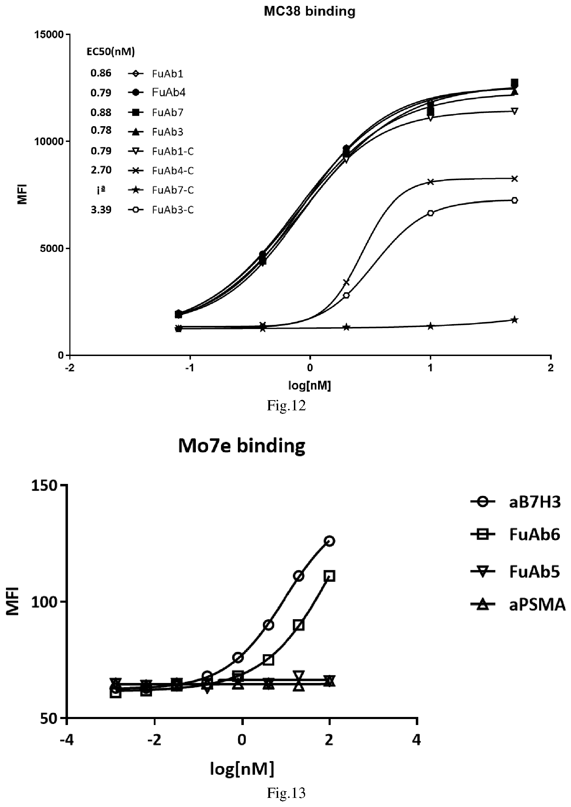

As shown in Figure 12, regarding the binding activity to MC38 cells, the flow cytometry binding curve of digested sample FuAb1-C of fusion protein FuAb1 without cleavage site shows no obvious change compared with that of FuAb1 sample. The flow cytometry binding curves of fusion proteins with a single cleavage site (e.g., FuAb4, FuAb3) shift to the right after digestion with decreased binding activity, while fusion proteins with double cleavage sites (e.g., FuAb7) show no cell binding activity after digestion.

### 5.3 Mo7e Cell Surface Receptor Binding Activity Assay

Suspension human megakaryoblastic leukemia cells Mo7e (Mo7e are human IL-2Rβγ receptor-positive cells) were cultured. 2×10⁵ cells were aliquoted into each flow cytometry staining well, and blocked with pre-cooled 2% FBS-PBS blocking solution for 30 minutes. The cytokine fusion antibody sample was diluted to 100 nM with blocking solution, and serially diluted 5-fold from this starting concentration for a total of 8 gradients. 0 nM concentration was set as the staining background. 100 µL of diluted cytokine fusion antibody sample was added to each well and incubated at 4°C for 30 minutes. After washing 3 times with PBS, 100 µL of APC-labeled anti-human Fc monoclonal antibody diluted according to the instructions was added to each well, incubated at 4°C for 30 minutes, and washed 3 times with PBS. 200 µL of blocking solution was added to the washed flow tube, and the resuspended cell suspension was used for flow cytometry analysis. EC50 values were calculated using the "log(agonist) vs. response - Variable slope (four parameters)" analysis model of GraphPad Prism 7 software.

The results are shown in Figure 13. FuAb5 shows very weak binding activity to Mo7e, while FuAb6 shows strong binding activity to Mo7e, which is closely related to the expression of B7H3 receptor on Mo7e cells (commercial PE-labeled anti-human B7H3 antibody stains Mo7e cells positively, data not shown).

### Example 6. Cell Proliferation Activity Assays

Suspension human megakaryoblastic leukemia cells Mo7e and murine T lymphocytes CTLL2 (CTLL2 are cells expressing murine IL2αβγ receptor) were cultured. Cultured CTLL2 cells were centrifuged, washed 3 times with RPMI 1640 medium, and resuspended in 10% FBS-RPMI 1640 medium at a density of 2×10⁵ cells/mL. The fusion antibody sample was serially diluted 5-fold, 10 µL was added to a black clear-bottom 96-well culture plate, followed by 90 µL of resuspended cells, and mixed gently by tapping. The plate was left to stand and incubated in an incubator for 24 h. Lysis and reading were performed using an equal volume of CellTiter-Glo according to the manufacturer's instructions. Cultured Mo7e cells were centrifuged, washed 3 times with RPMI 1640 medium, and resuspended in 10% FBS-RPMI 1640 medium at a density of 2×10⁵ cells/mL. 10 µL of 5-fold serially diluted fusion antibody sample (digested and undigested) was added to a black clear-bottom 96-well culture plate, followed by 90 µL of resuspended cells, and mixed gently by tapping. The plate was left to stand and incubated in an incubator for 72 h. Lysis and reading were performed using an equal volume of CellTiter-Glo according to the manufacturer's instructions. EC50 values were calculated using the "log(agonist) vs. response - Variable slope (four parameters)" analysis model of GraphPad Prism 7 software.

PBMC cells co-activated with anti-human CD3 antibody and anti-human CD28 antibody were collected, centrifuged at 300 g for 5 minutes, the supernatant was discarded, washed 3 times with RPMI 1640 medium, and resuspended in 10% FBS-RPMI 1640 medium at a density of 2×10⁵ cells/mL. 10 µL of serially diluted fusion antibody sample was added to a black clear-bottom 96-well culture plate, followed by 90 µL of resuspended cells, and mixed gently by tapping. The plate was left to stand and incubated in an incubator for 72 h. Lysis and reading were performed using an equal volume of CellTiter-Glo according to the manufacturer's instructions. EC50 values were calculated using the "log(agonist) vs. response - Variable slope (four parameters)" analysis model of GraphPad Prism 7 software.

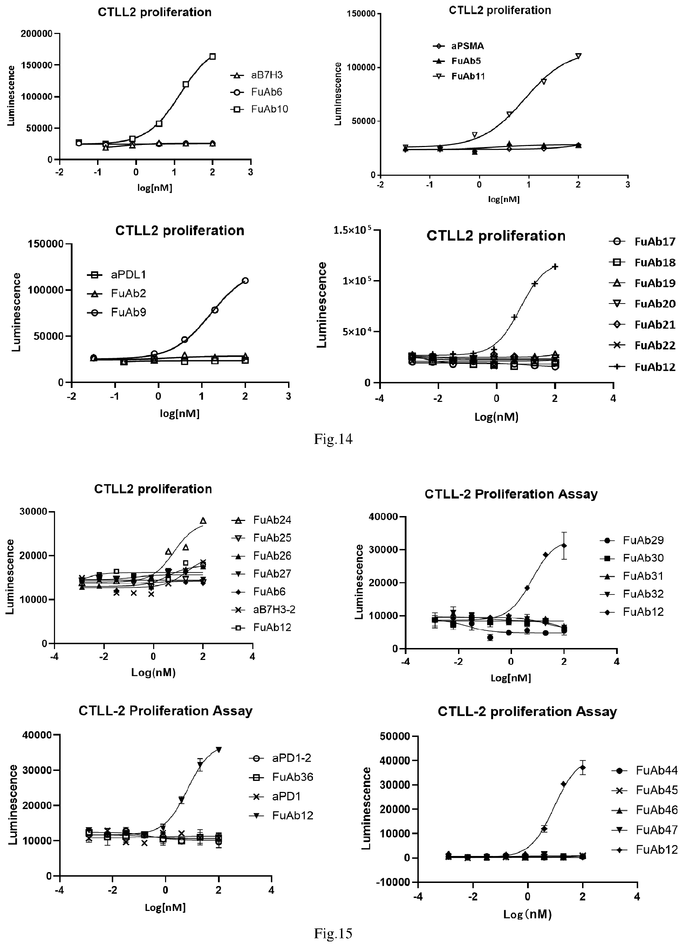

The results are shown in Figures 14-15. Compared with fusion proteins with IL15 and IL15Rα located at the N-termini of antibody variable regions (e.g., FuAb9, FuAb10, FuAb11 and FuAb12), fusion proteins with IL15 and IL15Rα located between antibody variable and constant regions (e.g., FuAb2, FuAb5, FuAb6, FuAb17-22, FuAb24-25, FuAb29-32, FuAb36 and FuAb44-47) show no obvious pro-proliferative activity on CTLL2 cells. It can be seen that the embedded fusion mode can greatly attenuate the downstream proliferation activity resulting from the interaction of the IL-15/IL-15Rα complex with its corresponding receptor.

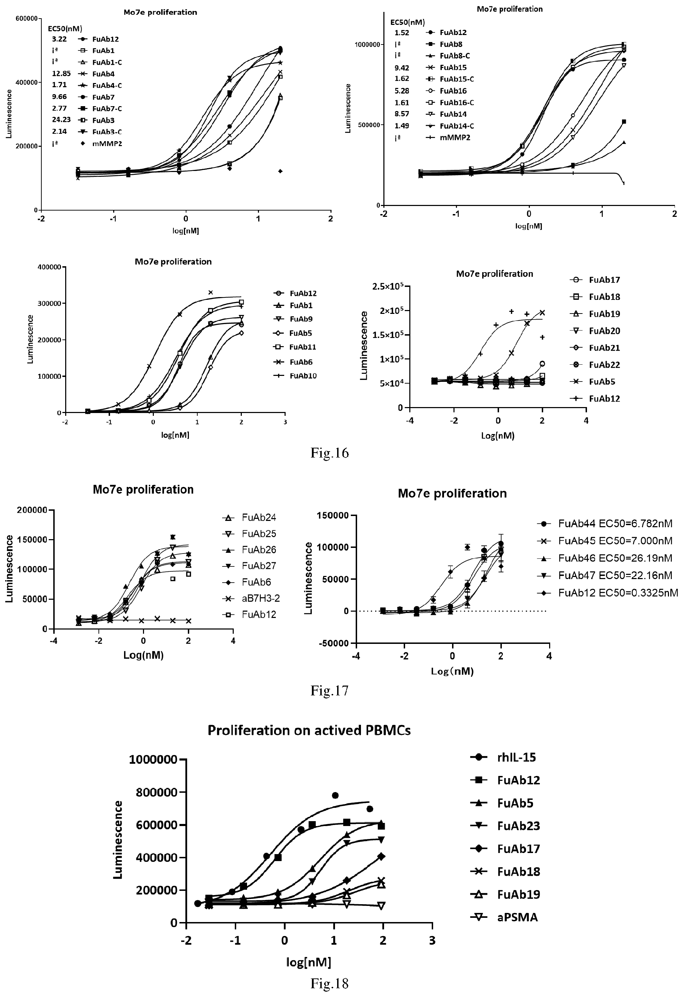

As shown in Figure 16, for Mo7e cells, compared to fusion proteins with IL15 and IL15Rα located at the N-termini of antibody variable regions (e.g., FuAb9, FuAb10, FuAb11 and FuAb12), the proliferation promoting activities of three embedded fusion antibodies FuAb1, FuAb5 and FuAb6 on Mo7e cells are reduced by 5 to 10-fold, which corresponds to the weak binding activity to IL-2βγ receptor shown in flow cytometry. In addition, the proliferation promoting ability of FuAb6 is significantly stronger than that of FuAb1 and FuAb5, presumably related to the expression of B7H3 antigen on the surface of Mo7e cells (flow cytometry results not shown), indicating that the binding of FuAb6 to human B7H3 receptor on the surface of Mo7e cells has a synergistic promoting effect on the proliferation signal of the cytokine complex.

As shown in Figure 17, the proliferation promoting activities of embedded fusion proteins obtained by fusion with aPD1, aPD1-2, aPD1-3 antibodies (FuAb44-47) on Mo7e cells are reduced by more than 20-100-fold compared to FuAb12. In addition, compared to the positive control sample FuAb12 (IL-15 and IL-15Rα fused to the N-termini of antibody heavy and light chains, respectively), the proliferation activities of FuAb24, 25, 26, 27 on Mo7e cells are increased by 5-10-fold, indicating that the binding of FuAb6 to human B7H3 receptor on the surface of Mo7e cells has a synergistic promoting effect on the proliferation signal of the cytokine complex.

As shown in Figure 16, regarding the promotion of Mo7e proliferation activity, the proliferation activities increase sequentially for fusion proteins without cleavage site (e.g., FuAb1, FuAb8 and FuAb17-22), fusion proteins with a single cleavage site (e.g., FuAb3, FuAb4, FuAb14 and FuAb15), and fusion protein samples with double cleavage sites (e.g., FuAb7 and FuAb16). Among them, the proliferation curves of digested fusion proteins without cleavage site are similar to those of undigested ones. The proliferation curves of fusion proteins with cleavage sites (e.g., FuAb4, FuAb3, FuAb14, FuAb15, FuAb7, FuAb16) shift to the left after digestion, and the proliferation activity restored to the same level as FuAb12. This may be due to the cleavage by MMP enzymes naturally released by megakaryocytic cells upon incubation [Blood 2011, 118(7): 1903-1911], leading to the release of the activity of chimeric IL15-IL15Rα.

Regarding the proliferation activity on PBMCs, the results are shown in Figure 18. The backbone antibody aPSMA has no effect on cell proliferation. Compared to the rhIL15 molecule alone and fusion protein with IL15 and IL15Rα located at the N-termini of antibody variable regions (FuAb12), the proliferation promoting activities of five embedded fusion antibodies (FuAb5, FuAb17, FuAb18, FuAb19, FuAb23) on PBMCs are reduced by 10-150-fold.

As shown in Figure 19, in the NK92 proliferation assay, chimeric fusion proteins (FuAb6, FuAb24-27, FuAb29-31, FuAb36, FuAb44-47) all exhibited relatively weak NK92 proliferation-promoting ability, with EC50 values more than 5-20-fold higher than that of sample FuAb12 (IL-15 and IL-15Rα fused to the N-termini of antibody heavy and light chains, respectively).

### Example 7. Efficacy and Toxicity in the MC38 Mouse Tumor Model

### 1. Fusion Antibody Targeting PDL1

A tumor model was established using murine colon cancer cells MC38 to evaluate the tumor growth inhibitory activity and body weight-related drug toxicity of the cytokine fusion antibody. Cell lines with antibody-targeted antigens on the surface were cultured, wherein MC38 cells naturally express murine PDL1 antigen on the surface and MMP-2 enzyme naturally exists in mice. The cells were adherently cultured in DMEM medium containing 10% FBS and harvested after trypsin digestion. C57BL6J mice aged 6-8 weeks were inoculated subcutaneously on the dorsal side with 5×10⁵ MC38 cells. When the tumor size reached a volume of 50-100 mm³, 2 mg/kg was administered via tail vein twice. Tumor volume of mice was recorded every two days during this period. Mice were euthanized when the tumor volume exceeded 1000 mm³.

The results are shown in Figure 20. Fusion antibodies with IL15 and IL15Rα located between antibody variable and constant regions (FuAb2 and FuAb8) show similar tumor inhibitory activity to fusion antibodies with IL15 and IL15Rα located at the N-termini of antibody variable regions (FuAb9 and FuAb12). Fusion proteins with cleavage sites (e.g., FuAb4, FuAb3, FuAb7) all show superior tumor inhibitory activity to aPDL1 mAb, and the tumor inhibitory activity is similar to that of FuAb12. Meanwhile, fusion proteins with cleavage sites are superior to FuAb12 in terms of body weight maintenance. It can be seen that the chimeric fusion mode can reduce IL15 activity, relying on N-terminal antigen-binding target enrichment and enzymatic cleavage to release IL-15 activity. This fusion method brings better tumor inhibitory activity to the fusion protein samples carrying cleavage sites, while greatly alleviating systemic toxicity manifestations related to body weight. Fusion proteins without a cleavage site, relying on strong N-terminal antigen-binding target enrichment and weak IL15 activity, also exhibited good tumor inhibitory activity without obvious body weight loss. The tumor inhibitory activities of aPDL1 monoclonal antibody, fusion protein without cleavage sites, and fusion protein with cleavage site increase sequentially, which is consistent with the mechanism that cleavage releases the activity of chimeric IL15-IL15Rα.

### 2. Fusion Antibody Targeting PD1

A tumor model was established using murine colon cancer cells MC38 to evaluate the tumor growth inhibitory activity and body weight-related drug toxicity of the cytokine fusion antibody. Cell lines carrying antibody-targeted antigens on the surface were cultured, wherein MC38 cells naturally express murine PDL1 antigen on the surface. The cells were adherently cultured in DMEM medium containing 10% FBS and harvested after trypsin digestion. C57BL6J mice aged 6-8 weeks were inoculated under the armpit with 5×10⁵ MC38 cells. When the tumor size reached a volume of 50-100 mm³, different doses were administered via tail vein. Tumor volume of mice was recorded every two days during this period. Mice were euthanized when the tumor volume exceeded 1000 mm³. Spleen samples of mice were collected for flow cytometry analysis of cell populations. Flow cytometry staining used CD45⁺CD3⁺ to label T cell populations, CD45⁺CD3⁺CD4⁺ to label CD4⁺ T cell populations, CD45⁺CD3⁺CD8⁺ to label CD8⁺ T cell populations, PD1⁺ to label PD1-positive cell populations, and Foxp3 to label Foxp3-positive cell populations.

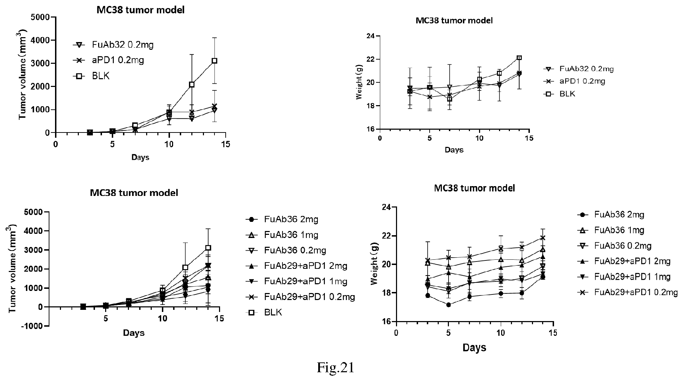

As shown in Figure 21, the tumor inhibitory effect of FuAb32 is superior to that of aPD1 monoclonal antibody. FuAb36 shows very weak tumor inhibitory activity, indicating that IL-15 fusion alone cannot achieve a good tumor inhibitory effect without PD1 targeting. The tumor inhibitory effect of FuAb29 combined with aPD1 mAb is similar to that of aPD1 and FuAb32, indicating that in the case of combination, IL15 fusion alone and aPD1 monoclonal antibody alone cannot produce good synergistic tumor inhibitory effect.

Body weight was not affected by administration throughout the observation period, no health-related discomfort was observed, and no mouse death occurred.

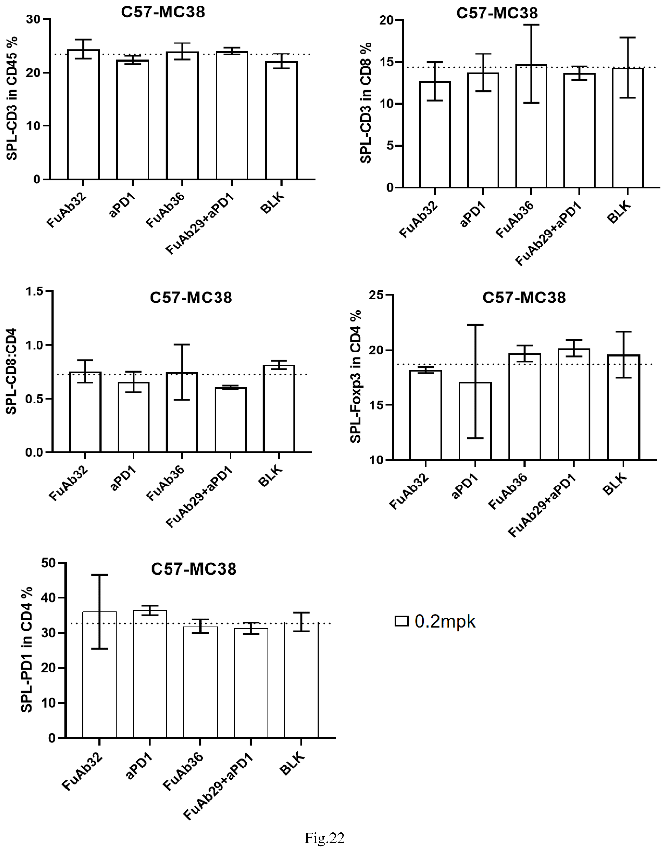

Flow cytometry staining results are shown in Figure 22. Samples in the 0.2 mg/kg dose group show no obvious changes in cell populations, indicating that each sample has little effect on the mobilization of immune cells in the mouse spleen system, suggesting no toxicity risk in the mouse system.

### Example 8. Influence of Drug Dose on Efficacy and Toxicity in the MC38 Model

A mouse colon carcinoma MC38 tumor model was established. 6-8 week old C57BL6J mice were inoculated subcutaneously on the back with 5x10⁵ MC38 cells. When the tumor size reached a volume of 50-100 mm³, doses of 2 mg/kg, 5 mg/kg, or 10 mg/kg were administered once via tail vein injection. Tumor volume changes were recorded every two days. Mice were euthanized when the tumor volume exceeded 1000 mm³.

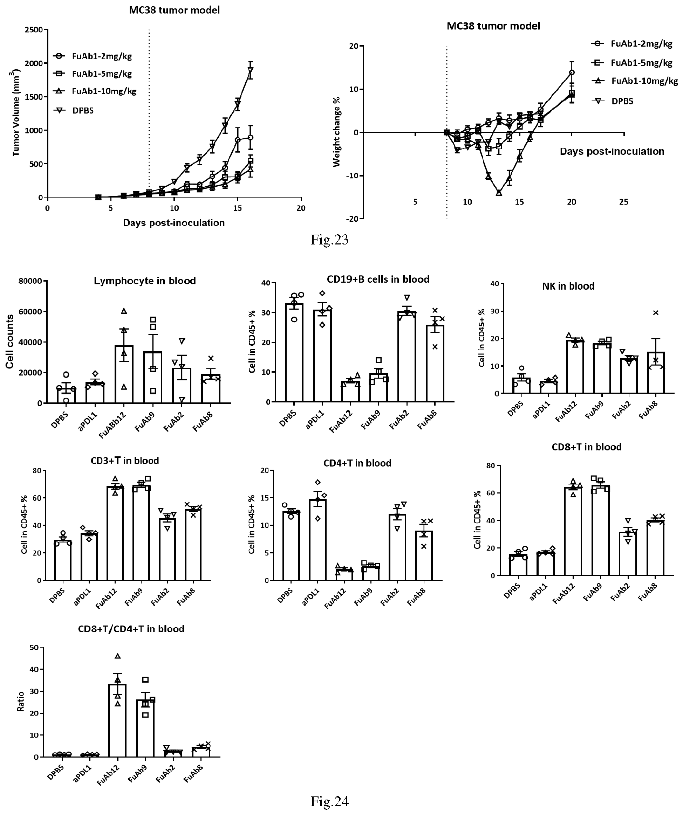

As shown in Figure 23, compared with the DPBS group, FuAb1 at 2 mg/kg, 5 mg/kg and 10 mg/kg all exhibits good tumor growth inhibitory activity under a single administration frequency. Among them, the body weight of mice in the 10 mg/kg administration group decreased significantly two days after administration, while the body weight of mice in the 2 mg/kg and 5 mg/kg dose groups was not affected by administration throughout the observation period, indicating that the fusion antibody has a good safe and effective dose window.

### Example 9. Mechanism of Drug Action in the MC38 Mouse Tumor Model

A tumor model was established using murine colon cancer cells MC38. C57BL6J mice aged 6-8 weeks were inoculated subcutaneously on the dorsal side with 5×10⁵ MC38 cells. When the tumor size reached a volume of 300-500 mm³, a single dose of 2 mg/kg was administered via tail vein. Mice were euthanized on the fourth day after administration, and peripheral blood samples, spleen samples and tumor tissue samples of mice were collected for flow cytometry analysis of cell populations.

Flow cytometry staining used CD45⁺CD3⁺ to label T cell populations, CD45⁺CD3⁺CD4⁺ to label CD4⁺ T cell populations, CD45⁺CD3⁺CD8⁺ to label CD8⁺ T cell populations, CD45⁺CD19⁺ to label B cell populations, and CD45⁺CD335⁺ to label NK cell populations.

The results are shown in Figures 24-26. In the analysis of peripheral blood samples, spleen samples and tumor-infiltrating lymphocytes (TILs), the performance of cell populations related to tumor inhibition and systemic toxicity can be demonstrated.

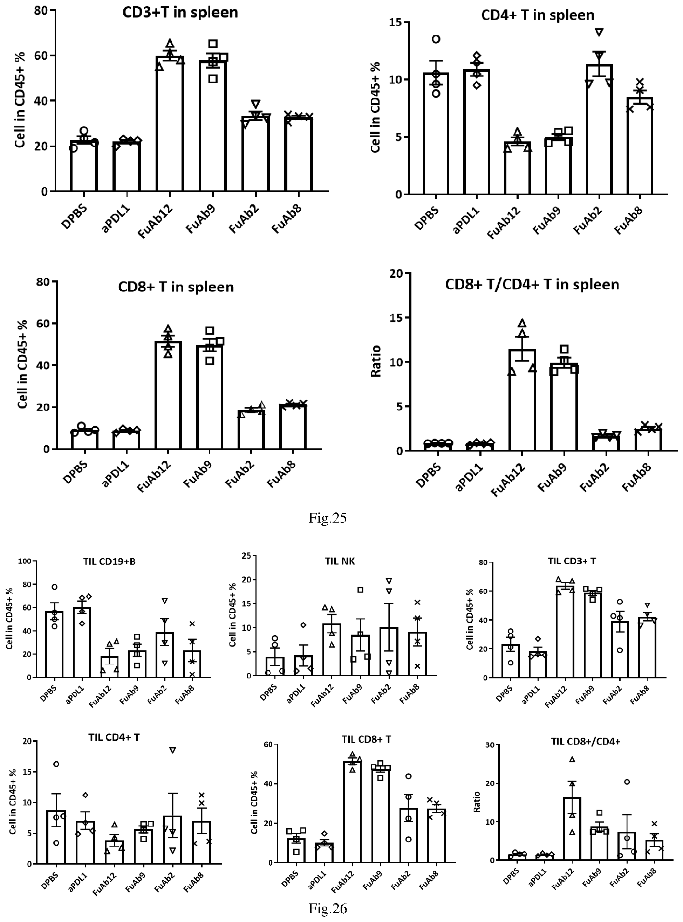

As shown in Figures 24 and 25, FuAb12/FuAb9 exhibit severe body weight-related systemic toxicity in Example 7. In cell population analysis, they show massive proliferation of NK/T lymphocytes in peripheral blood and spleen (non-tumor related). Compared with FuAb12/FuAb9, chimeric fusion proteins FuAb2/FuAb8 show smaller changes in cell populations in peripheral blood and spleen (non-tumor related), with the proportions of various cells similar to those in the PDL1 mAb group. It can be seen that the chimeric fusion protein can maintain the stability of cell populations in peripheral blood and spleen, effectively avoiding systemic toxicity caused by lymphocyte proliferation, which is consistent with the body weight changes shown in Example 7.

As shown in Figure 26, in the analysis of cell populations in the tumor environment, compared to the aPDL1 treatment group, the fusion protein treatment groups (FuAb12, FuAb9, FuAb2 and FuAb8) all show a significant increase in the proportion of killing-related lymphocytes (CD8⁺ T cells and NK cells). It can be seen that the chimeric fusion protein can achieve the therapeutic effect of tumor inhibition by specifically expanding killing-related lymphocytes in the tumor environment, which is consistent with the tumor inhibition results shown in Example 7.

In summary, only the chimeric fusion proteins FuAb2/FuAb8 can specifically increase the proportions of CD8⁺ T cells and NK cells in the tumor environment without affecting the cell populations in peripheral blood and spleen. Only the chimeric fusion proteins FuAb2/FuAb8 have the ability to reduce cellular toxicity in the systemic system, exert biological effects in the tumor microenvironment, enhance the activation of NK cells and CD8⁺ T cells in the tumor site tissue region, and achieve tumor growth inhibitory activity.

### Example 10. Influence of Drug Dose on Efficacy and Toxicity in the MC38-hB7H3 Mouse Tumor Model

A tumor model was established using murine colon cancer cells MC38-hB7H3 to evaluate the tumor growth inhibitory activity and body weight-related drug toxicity of the cytokine fusion antibody. MC38-hB7H3 cells were adherently cultured in DMEM medium containing 10% FBS and harvested after trypsin digestion. C57BL6J mice aged 6-8 weeks were inoculated subcutaneously on the dorsal side with 5×10⁵ MC38-hB7H3 cells. When the tumor size reached a volume of 50-100 mm³, doses of 2 mg/kg, 5 mg/kg, 10 mg/kg were administered via tail vein, for a total of two doses. Tumor volume of mice was recorded every two days during this period. Mice were euthanized when the tumor volume exceeded 1000 mm³.

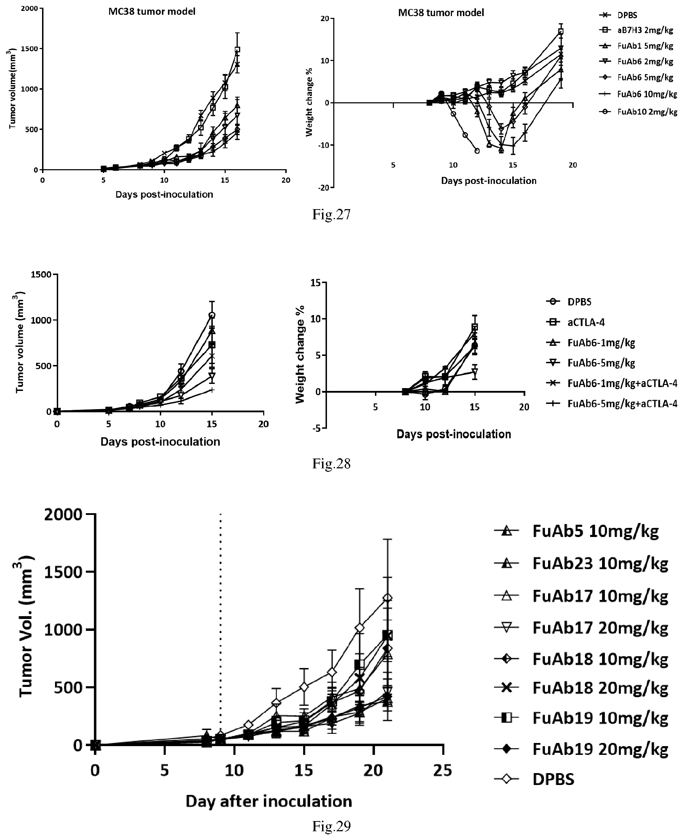

The results are shown in Figure 27. From days10-15 post-inoculation, the tumor volume in the DPBS-treated group exceeded 1000 mm³. Compared to the PBS group, the monoclonal antibody against target antigen aB7H3 at a dose of 2 mg/kg dose no exhibit significant tumor growth inhibitory activity. The fusion protein FuAb10 with IL15 and IL15Rα located at the N-termini of antibody variable regions exhibits significant tumor inhibitory activity, but its body weight decreased by more than 20% between days 10-15. In contrast, FuAb6 at doses of 2 mg/kg, 5 mg/kg and 10 mg/kg all have significant tumor growth inhibitory activity, and the body weight of mice is less affected by treatment throughout the observation period. The body weight loss trend in the 10 mg/kg FuAb6 treatment group is significantly improved compared to that of 2mg/kg FuAb10 dose group, indicating that FuAb6 has a good safe and effective dose window.

As shown in Figure 28, similar conclusions were reached when combined with the immune checkpoint inhibitor aCTLA-4: the fusion antibody with IL-15 and IL-15Rα located between the variable and constant regions shows good efficacy and safety, with a large safe and effective dose window (2-10 mg/kg both safe and effective).

### Example 11. Pharmacokinetic Evaluation in Mice

6-8 week old C57BL6J mice were administered via tail vein at doses of 2 mg/kg or 5 mg/kg, respectively. Blood was collected from the retro-orbital sinus at different time points. The concentration of intact fusion antibody in serum samples at each time point was detected by ELISA, and the data were processed by GraphPad Prism.

The results are shown in Table 3 below. FuAb2, FuAb6, FuAb17, FuAb19 and FuAb23 all show pharmacokinetic performance similar to antibody molecules, indicating that the embedded fusion mode endows the fusion antibody molecule with a good serum half-life.

**Table 3: PK parameters of the fusion antibodies**

| Fusion antibody | Dose | t1/2 (hr) | Cmax (ng/ml) |
|---|---|---|---|
| FuAb6 | 2mg/kg | 188.07 | 35255 |
| | 5mg/kg | 100.30 | 99615 |
| FuAb2 | 2mg/kg | 30.70 | 29860 |
| | 5mg/kg | 31.02 | 95286 |
| aPDL1 | 5mg/kg | 24.87 | 102176 |
| aPSMA | 5mg/kg | 188.07 | 145890 |
| FuAb17 | 5mg/kg | 158.37 | 131720 |
| FuAb19 | 5mg/kg | 125.38 | 147884 |
| FuAb23 | 5mg/kg | 177.01 | 141046 |

### Example 12. Efficacy and Toxicity in the MC38-hPSMA Mouse Tumor Model

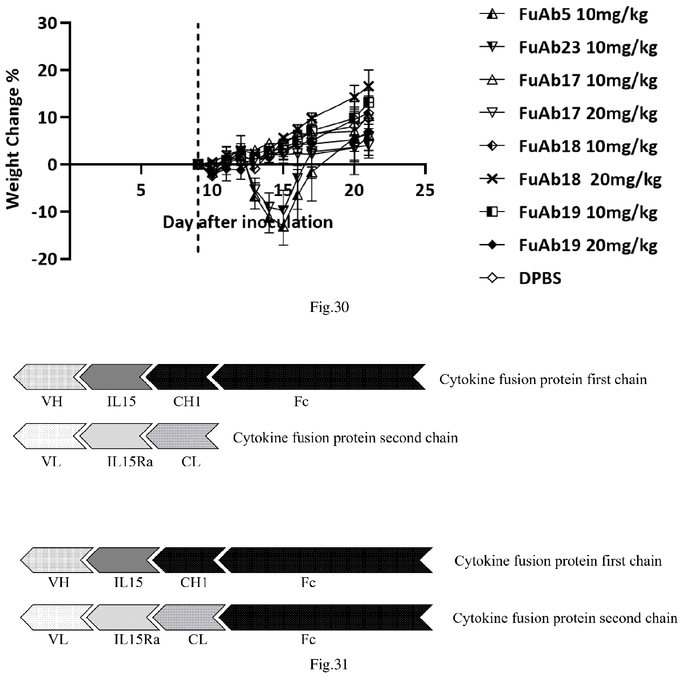

A tumor model was established using murine colon cancer cells MC38-hPSMA to evaluate the tumor growth inhibitory activity and body weight-related drug toxicity of the cytokine fusion antibody. Cell lines carrying antibody target antigens on the surface were cultured, wherein MC38-hPSMA cells naturally express murine PDL1 antigen and heterologously expressed human PSMA antigen on the surface. The cells were adherently cultured in DMEM medium containing 10% FBS and harvested after trypsin digestion. 6-8 week old C57BL6J mice were inoculated subcutaneously in the axilla with 5×10⁵ MC38-hPSMA cells. When the tumor size reached a volume of 50-100 mm³, different doses were administered via tail vein. Tumor volume of mice was recorded every two days during this period. Mice were euthanized when the tumor volume exceeded 1000 mm³. The results are shown in Figures 29-30. From days 17-19 post-inoculation, the tumor volume of the DPBS-treated group exceeded 1000 mm³, while FuAb17-19 all show good tumor inhibitory activity and safety.

From the above description, it can be seen that the above embodiments of the present invention achieve the following technical effects: utilizing the symmetric Y-shaped architecture of natural antibodies, using VH-IL-15-CH1-Fc fragment or VH-IL-15Rα-CH1-Fc fragment as the first chain, and VL-IL-15Rα-CL fragment, VL-IL-15-CL-Fc fragment, VL-IL-15-CL fragment or VL-IL-15Rα-CL-Fc fragment as the second chain, the resulting fusion antibody posses stability and half-life similar to natural antibodies. Furthermore, it significantly reduces the activity of IL15/IL15Rα complex, thereby greatly reducing systemic toxicity. Leveraging the targeting capability of the antibody allows it to function specifically within the tumor microenvironment, enhancing the cellular targeting of the IL-15/IL-15Rα complex. In addition, as no exogenous sequence is introduced, the immunogenicity risk is minimized, which improves the productivity and developability of the molecular architecture itself.

The above descriptions are only preferred embodiments of the present invention and are not intended to limit the present invention. For those skilled in the art, the present invention may have various modifications and variations. Any modification, equivalent replacement, improvement, etc. made within the spirit and principle of the present invention shall be included in the protection scope of the present invention.

## Claims

1. A fusion antibody, comprising: a) IL-15; b) IL-15Ra; and c) an antibody targeting a therapeutically relevant cell-surface antigen, the antibody comprising a heavy-chain variable region VH, a heavy-chain constant region CH1, a light-chain variable region VL, and a light-chain constant region CL; wherein IL-15 is located between the C-terminus of VH and the N-terminus of CH1 and IL-15Ra is located between the C-terminus of VL and the N-terminus of CL; or IL-15 is located between the C-terminus of VL and the N-terminus of CL and IL-15Ra is located between the C-terminus of VH and the N-terminus of CH1.

2. The fusion antibody according to claim 1, wherein IL-15 is directly fused or fused through a linker peptide between the C-terminus of VH and the N-terminus of CH1, and IL-15Ra is directly fused or fused through a linker peptide between the C-terminus of VL and the N-terminus of CL; or IL-15 is directly fused or fused through a linker peptide between the C-terminus of VL and the N-terminus of CL, and IL-15Ra is directly fused or fused through a linker peptide between the C-terminus of VH and the N-terminus of CH1.

3. The fusion antibody according to claim 1 or 2, wherein the C-terminus of CH1 contains an Fc fragment, and the C-terminus of CL contains or does not contain an Fc fragment.

4. The fusion antibody according to claim 1, wherein IL-15 has the amino acid sequence shown in SEQ ID NO:12 or SEQ ID NO:81, or an amino acid sequence having at least 80%, preferably at least 90%, more preferably at least 95% homology thereto.

5. The fusion antibody according to claim 1, wherein IL-15Ra is full-length IL-15Ra or the Sushi domain of IL-15Ra; preferably IL-15Ra has the amino acid sequence shown in SEQ ID NO:13 or SEQ ID NO:14, or an amino acid sequence having at least 80%, preferably at least 90%, more preferably at least 95% homology thereto.

6. The fusion antibody according to any one of claims 1-5, wherein the therapeutically relevant cell-surface antigen is an immune checkpoint protein or a tumor antigen.

7. The fusion antibody according to claim 6, wherein the therapeutically relevant cell-surface antigen is any one of the following: PD1, PDL1, B7H3, PSMA, Nectin-4, CD19, BCMA, CD22, CD20, GPCR5D, CD21, CD81, CD40, CD79, CD80, CD86, ICAM-1 (CD54), CD11a, CD18, CD45, GPC3, HER2, EGFR, GCN4, Tim3, CLL1, Trop2, Claudin18.2, Claudin6, Muc1, Muc16, or GIST.

8. The fusion antibody according to claim 7, wherein the antibody targeting the therapeutically relevant cell-surface antigen comprises an anti-PDL1 antibody having the heavy-chain CDR sequences HCDR1, HCDR2 and HCDR3 shown in SEQ ID NOs:15-17 and the light-chain CDR sequences LCDR1, LCDR2 and LCDR3 shown in SEQ ID NOs:18-20; or a VH sequence shown in SEQ ID NO:21 and a VL sequence shown in SEQ ID NO:22; or the heavy-chain amino acid sequence shown in SEQ ID NO:23 and the light-chain amino acid sequence shown in SEQ ID NO:24;
preferably, the antibody targeting the therapeutically relevant cell-surface antigen comprises an anti-B7H3 antibody having HCDR1, HCDR2, HCDR3 shown in SEQ ID NOs: 25-27 and LCDR1, LCDR2, LCDR3 shown in SEQ ID NOs: 28-30; or a VH sequence shown in SEQ ID NO: 31 or 118 and a VL sequence shown in SEQ ID NO: 32 or 119; or a heavy-chain amino acid sequence shown in SEQ ID NO: 33 and a light-chain amino acid sequence shown in SEQ ID NO: 34;
preferably, the antibody targeting the therapeutically relevant cell-surface antigen comprises an anti-PSMA antibody having HCDR1, HCDR2, HCDR3 shown in SEQ ID NOs: 35-37 and LCDR1, LCDR2, LCDR3 shown in SEQ ID NOs: 38-40; or a VH sequence shown in SEQ ID NO: 41 and a VL sequence shown in SEQ ID NO: 42; or a heavy-chain amino acid sequence shown in SEQ ID NO: 43 and a light-chain amino acid sequence shown in SEQ ID NO: 44;
preferably, the antibody targeting the therapeutically relevant cell-surface antigen comprises an anti-PD1 antibody having HCDR1, HCDR2, HCDR3 shown in SEQ ID NOs: 120-122, SEQ ID NOs: 126-128, or SEQ ID NOs: 132-134, and LCDR1, LCDR2, LCDR3 shown in SEQ ID NOs: 123-125, SEQ ID NOs: 129-131, or SEQ ID NOs: 135-137; or a VH sequence shown in SEQ ID NO: 138 and a VL sequence shown in SEQ ID NO: 139; or a heavy-chain amino acid sequence shown in SEQ ID NO: 140 or 142 and a light-chain amino acid sequence shown in SEQ ID NO: 141 or 143.

9. The fusion antibody according to claim 7 or 8, wherein the antibody targeting the therapeutically relevant cell-surface antigen comprises an Fc fragment; preferably the Fc fragment is selected from human IgG1, IgG2, IgG3, or IgG4 Fc fragments.

10. The fusion antibody according to claim 2, wherein the linker peptide is a cleavable linker peptide or a non-cleavable linker peptide; preferably each linker peptide is independently selected from [GGGGS]n, where n is 1-6, EAAAK (SEQ ID NO:79), EAAAKEAAAK (SEQ ID NO:80), GGGGSIPVSLRSGGGGGSG (SEQ ID NO:65), GGGGSIPVSLRSGGGSG (SEQ ID NO:62), GGGGSG (SEQ ID NO:145), GGGGSGGGGSG (SEQ ID NO:146), or GGGGSGGGGSGGGGSG (SEQ ID NO:64);
more preferably, the linker is GGGGS (SEQ ID NO: 144), GGGGSG (SEQ ID NO: 145), GGGGSGGGGS (SEQ ID NO: 63), GGGGSGGGGSG (SEQ ID NO: 146), GGGGSGGGGSGGGGSG (SEQ ID NO: 64), GGGGSIPVSLRSGGGGGSG (SEQ ID NO: 65), or GGGGSIPVSLRSGGGSG (SEQ ID NO: 62).

11. The fusion antibody according to any one of claims 1-5, wherein the fusion antibody comprises a heavy chain and a light chain having any one of the amino acid sequence pairs listed in the following: 1)SEQ ID NO: 1 and SEQ ID NO: 2; 2) SEQ ID NO: 3 and SEQ ID NO: 4; 3) SEQ ID NO: 1 and SEQ ID NO: 5; 4) SEQ ID NO: 1 and SEQ ID NO: 6; 5) SEQ ID NO: 7 and SEQ ID NO: 8; 6) SEQ ID NO: 9 and SEQ ID NO: 10; 7) SEQ ID NO: 5 and SEQ ID NO: 11; 8) SEQ ID NO: 45 and SEQ ID NO: 46; 9) SEQ ID NO: 57 and SEQ ID NO: 58; 10) SEQ ID NO: 59 and SEQ ID NO: 60; 11) SEQ ID NO: 57 and SEQ ID NO: 60; 12) SEQ ID NO: 67 and SEQ ID NO: 68; 13) SEQ ID NO: 69 and SEQ ID NO: 70; 14) SEQ ID NO: 71 and SEQ ID NO: 72; 15) SEQ ID NO: 73 and SEQ ID NO: 74; 16) SEQ ID NO: 75 and SEQ ID NO: 76; 17) SEQ ID NO: 77 and SEQ ID NO: 78; 18) SEQ ID NO: 82 and SEQ ID NO: 8; 19) SEQ ID NO: 83 and SEQ ID NO: 84; 20) SEQ ID NO: 85 and SEQ ID NO: 84; 21) SEQ ID NO: 83 and SEQ ID NO: 86; 22) SEQ ID NO: 85 and SEQ ID NO: 86; 23) SEQ ID NO: 88 and SEQ ID NO: 89; 24) SEQ ID NO: 90 and SEQ ID NO: 91; 25) SEQ ID NO: 92 and SEQ ID NO: 93; 26) SEQ ID NO: 94 and SEQ ID NO: 95; 27) SEQ ID NO: 96 and SEQ ID NO: 97; 28) SEQ ID NO: 98 and SEQ ID NO: 99; 29) SEQ ID NO: 102 and SEQ ID NO: 103; 30) SEQ ID NO: 106 and SEQ ID NO: 107; 31) SEQ ID NO: 108 and SEQ ID NO: 107; 32) SEQ ID NO: 112 and SEQ ID NO: 113; 33) SEQ ID NO: 112 and SEQ ID NO: 114; 34) SEQ ID NO: 115 and SEQ ID NO: 116; 35) SEQ ID NO: 115 and SEQ ID NO: 117.

12. A DNA molecule encoding the fusion antibody according to any one of claims 1-11.

13. A recombinant plasmid linked to the DNA molecule according to claim 12.

14. A host cell transformed with the recombinant plasmid according to claim 13.

15. The host cell according to claim 14, wherein the host cell comprises a prokaryotic cell or a eukaryotic cell.

16. Use of the fusion antibody according to any one of claims 1-11 in the preparation of a medicament for preventing and/or treating cancer.

17. The use according to claim 16, wherein the cancer is any one of lung cancer, melanoma, colon cancer, rectal cancer, liver cancer, breast cancer, lymphoma, prostate cancer, or a hematologic tumor.

18. A medicament comprising the fusion antibody according to any one of claims 1-11.

19. The medicament according to claim 18, further comprising an immune checkpoint inhibitor used in combination with the fusion antibody, wherein the immune checkpoint inhibitor is any one or more of aPDL1, aCTLA4, a41BB, or aLAG3.
